(19) Europäisches Patentamt
European Patent Office
Office européen des brevets

(11) EP 1 713 791 B1

(12) **EUROPEAN PATENT SPECIFICATION**

(45) Date of publication and mention of the grant of the patent:
**30.04.2008 Bulletin 2008/18**

(21) Application number: **04813689.9**

(22) Date of filing: **13.12.2004**

(51) Int Cl.:
***C07D 401/04*** (2006.01) ***C07D 213/84*** (2006.01)
***A61K 31/4439*** (2006.01) ***A61P 25/04*** (2006.01)

(86) International application number:
**PCT/US2004/041401**

(87) International publication number:
**WO 2005/066155 (21.07.2005 Gazette 2005/29)**

(54) **5-FLUORO- AND CHLORO-PYRIDIN-2-YL-TETRAZOLES AS LIGANDS OF THE METABOTROPIC GLUTAMATE RECEPTOR-5**

5-FLUOR- UND CHLOR-PYRIDIN-2-YL-TETRAZOLE ALS LIGANDEN DES METABOTROPEN GLUTAMATREZEPTORS-5

5-FLUORO- ET CHLORO-PYRIDIN-1-YL-TETRAZOLES UTILISES EN TANT QUE LIGANDS DU RECEPTEUR METABOTROPIQUE DU GLUTAMATE DE TYPE 5

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **19.12.2003 US 530633 P**

(43) Date of publication of application:
**25.10.2006 Bulletin 2006/43**

(73) Proprietor: **AstraZeneca AB**
**151 85 Södertälje (SE)**

(72) Inventors:
- **WENSBO, David,**
**AstraZeneca R & D Headquaters**
**SE-151 85 Södertälje (SE)**
- **EDWARDS, Louise**
**Mississauga, Ontario L5H 3S6 (CA)**
- **ISAAC, Methvin**
**c/o NPS Allelix Corporation**
**Toronto, Ontario M5G 1L8 (CA)**
- **MCLEOD, Donald A.**
**Salt Lake city, Utah 84121 (US)**
- **SLASSI, Abdelmalik**
**Mississauga, Ontario L5M 7J7 (CA)**
- **XIN, Tao**
**Woodbridge, Ontario L4H 2B1 (CA)**
- **STORMANN, Thomas M.**
**Salt Lake City, Utah 84105 (US)**

(74) Representative: **Plougmann & Vingtoft A/S**
**Sundkrogsgade 9**
**2100 Copenhagen (DK)**

(56) References cited:
**WO-A-02/068417**
**WO-A-03/029210**
**US-A1- 2003 055 085**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 713 791 B1

## Description

FIELD OF THE INVENTION

The present invention relates to new compounds, to pharmaceutical compositions containing said compounds and to the use of said compounds in therapy. The present disclosure further relates to processes for the preparation of said compounds.

BACKGROUND OF THE INVENTION

Glutamate is the major excitatory neurotransmitter in the mammalian central nervous system (CNS). Glutamate produces its effects on central neurons by binding to and thereby activating cell surface receptors. These receptors have been divided into two major classes, the ionotropic and metabotropic glutamate receptors, based on the structural features of the receptor proteins, the means by which the receptors transduce signals into the cell, and pharmacological profiles.
The metabotropic glutamate receptors (mGluRs) are G protein-coupled receptors that activate a variety of intracellular second messenger systems following the binding of glutamate. Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyil cyclase; increases in the formation of cyclic guanosine monophosphate (cGMP); activation of phospholipase $A_2$; increases in arachidonic acid release; and increases or decreases in the activity of voltage- and ligand-gated ion channels. Schoepp et al., Trends Phannacol. Sci. 14:13 (1993), Schoepp, Neurochem. Int. 24:439 (1994), Pin et al., Neuropharmacology 34:1 (1995), Bordi and Ugolini, Prog. Neurobiol. 59:55 (1999).
Eight distinct mGluR subtypes, termed mGluR1 through mGluR8, have been identified by molecular cloning. Nakanishi, Neuron 13:1031 (1994), Pin et al., Neuropharmacology 34:1 (1995), Knopfel et al., J. Med. Chem. 38:1417 (1995). Further receptor diversity occurs via expression of alternatively spliced forms of certain mGluR subtypes. Pin et al., PNAS 89:10331 (1992), Minakami et al., BBRC 199:1136 (1994), Joly et al., J. Neurosci. 15:3970 (1995).
Metabotropic glutamate receptor subtypes may be subdivided into three groups, Group I, Group II, and Group III mGluRs, based on amino acid sequence homology, the second messenger systems utilized by the receptors, and by their pharmacological characteristics, mGluR5 comprises mGluR1, mGluR5 and their alternatively spliced variants. The binding of agonists to these receptors results in the activation of phospholipase C and the subsequent mobilization of intracellular calcium.

### Neurological, psychiatric and pain disorders.

Attempts at elucidating the physiological roles of Group I mGluRs suggest that activation of these receptors elicits neuronal excitation. Various studies have demonstrated that Group I mGluRs agonists can produce postsynaptic excitation upon application to neurons in the hippocampus, cerebral cortex, cerebellum, and thalamus, as well as other CNS regions. Evidence indicates that this excitation is due to direct activation of postsynaptic mGluRs, but it also has been suggested that activation of presynaptic mGluRs occurs, resulting in increased neurotransmitter release. Baskys, Trends Pharmacol. Sci. 15:92 (1992), Schoepp, Neurochem. Int. 24:439 (1994), Pin et al., Neuropharmacology 34:1 (1995), Watkins et al., Trends Pharmacol. Sci. 15:33 (1994).
Metabotropic glutamate receptors have been implicated in a number of normal processes in the mammalian CNS. Activation of mGluRs has been shown to be required for induction of hippocampal long-term potentiation and cerebellar long-term depression. Bashir et al., Nature 363:347 (1993), Bortolotto et al., Nature 368:740 (1994), Aiba et al., Cell 79: 365 (1994), Aiba et al., Cell 79:377 (1994). A role for mGluR activation in nociception and analgesia also has been demonstrated. Meller et al., Neuroreport 4: 879 (1993), Bordi and Ugolini, Brain Res. 871:223 (1999). In addition, mGluR activation has been suggested to play a modulatory role in a variety of other normal processes including synaptic transmission, neuronal development, apoptotic neuronal death, synaptic plasticity, spatial learning, olfactory memory, central control of cardiac activity, waking, motor control and control of the vestibulo-ocular reflex. Nakanishi, Neuron 13: 1.031 (1994), Pin et al., Neuropharmacology 34:1, Knopfel et al., J. Med. Chem. 38:1417 (1995).
Further, Group I metabotropic glutamate receptors have been suggested to play roles in a variety of acute and chronic pathophysiological processes and disorders affecting the CNS. These include stroke, head trauma, anoxic and ischemic injuries, hypoglycemia, epilepsy, neurodegenerative disorders such as Alzheimer's disease, psychiatric disorders and pain. Schoepp et al., Trends Pharmacol. Sci. 14:13 (1993), Cunningham et al., Life Sci. 54:135 (1994), Hollman et al., Ann. Rev. Neurosci. 17:31 (1994), Pin et al., Neuropharmacology 34:1 (1995), Knopfel et al., J. Med. Chem. 38:1417 (1995), Spooren et al., Trends Pharmacol. Sci. 22:331 (2001), Gasparini et al. Curr. Opin. Pharmacol. 2:43 (2002),

Neugebauer Pain 98:1 (2002). Much of the pathology in these conditions is thought to be due to excessive glutamate-induced excitation of CNS neurons. Because Group I mGluRs appear to increase glutamate-mediated neuronal excitation via postsynaptic mechanisms and enhanced presynaptic glutamate release, their activation probably contributes to the pathology. Accordingly, selective antagonists of mGluR5 receptors could be therapeutically beneficial in all conditions underlain by excessive glutamate-induced excitation of CNS neurons, specifically as neuroprotective agents, analgesics or anticonvulsants.

Recent advances in the elucidation of the neurophysiological roles of metabotropic glutamate receptors generally and Group I in particular, especially mGluR5 receptors, have established these receptors as promising drug targets in the therapy of acute and chronic neurological and psychiatric disorders and chronic and acute pain disorders.

**Gastro intestinal disorders**

The lower esophageal sphincter (LES) is prone to relaxing intermittently. As a consequence, fluid from the stomach can pass into the esophagus since the mechanical barrier is temporarily lost at such times, an event hereinafter referred to as "reflux".

Gastro-esophageal reflux disease (GERD) is the most prevalent upper gastrointestinal tract disease. Current pharmacotherapy aims at reducing gastric acid secretion, or at neutralizing acid in the esophagus. The major mechanism behind reflux has been considered to depend on a hypotonic lower esophageal sphincter. However, e.g. Holloway & Dent (1990) Gastroenterol. Clin. N. Amer. 19, pp. 517-535, has shown that most reflux episodes occur during transient lower esophageal sphincter relaxations (TLESRs), i.e. relaxations not triggered by swallows. It has also been shown that gastric acid secretion usually is normal in patients with GERD.

The novel compounds according to the present invention are assumed to be useful for the inhibition of transient lower esophageal sphincter relaxations (TLESRs) and thus for treatment of gastro-esophageal reflux disorder (GERD).

The wording "TLESR", transient lower esophageal sphincter relaxations, is herein defined in accordance with Mittal, R.K, Holloway, R.H., Penagini, R., Blackshaw, L.A., Dent, J., 1995; Transient lower esophageal sphincter relaxation. Gastroenterology 109, pp. 601-610.

The wording "reflux" is herein defined as fluid from the stomach being able to pass into the esophagus, since the mechanical barrier is temporarily lost at such times.

The wording "GERD", gastro-esophageal reflux disease, is herein defined in accordance with van Heerwarden, M.A., Smout A.J.P.M., 2000; Diagnosis of reflux disease. Baillière's Clin. Gastroenterol. 14, pp. 759-774.

Because of their physiological and pathophysiological significance, there is a need for new potent mGluR agonists and antagonists that display a high selectivity for mGluR subtypes, particularly the Group I receptor subtype, such as the mGluR5 receptor.

**Prior art**

There are many properties of a compound that are of importance in determining its suitability as a drug product. Of high importance is the interaction of the compound at the target protein and its pharmacokinetic profile, i.e. its ability to access the target protein in sufficient quantity for a sufficient duration of time to have the desired therapeutic effect. The object of the present invention is to provide novel 5-substituted pyridines with surprisingly improved characteristics when compared to similarly substituted compounds, carrying other substituents at the 5-position of the pyridine ring as previously described. WO 03/077918 and WO 03/029210 describe 2-(5-tetrazolyl)-5-substituted pyridines exhibiting activity at the mGluR5 receptor. We have found that the appropriate 5-substituent on the pyridines has led to dramatic improvements over other substituents at that same position, when comparing both efficacy and pharmocokinetic profile, as measured in the laboratory.

SUMMARY OF THE INVENTION

The present invention relates to compounds of formula I

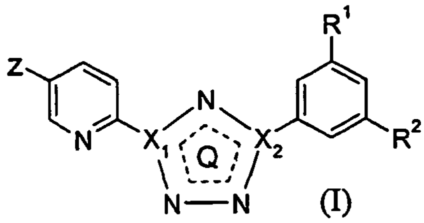

(I)

wherein:

$X_1$ is N and $X_2$ is C or $X_1$ is C and $X_2$ is N;
Z is fluoro or chloro;
$R^1$ and $R^2$ are independently selected from the group consisting of hydrogen, hydroxy, halo, $-C_{1-6}$alkylhalo, $-OC_{1-6}$alkylhalo, $-C_{1-6}$alkyl, $-OC_{0-6}$alkyl, $-C_{1-6}$alkylOR$^4$, $-OC_{2-6}$alkylOR$^4$, $-C_{0-6}$alkylcyano, $-C_{0-6}$alkylNR$^4$R$^5$ and $-OC_{2-6}$alkylNR$^4$R$^5$;
$R^4$ and $R^5$ are independently selected from the group consisting of hydrogen, and $-C_{1-3}$alkyl;

or salts, solvates or solvated salts thereof.

In another aspect of the invention there is provided the compound of formula I for use in therapy, especially for the treatment of mGluR5 receptor mediated disorders, and for the treatment of neurological disorders, psychiatric disorders, gastrointestinal disorders and pain disorders.

In a further aspect of the invention there is provided pharmaceutical compositions comprising a therapeutically effective amount of the compound of formula I in association with one or more pharmaceutically acceptable diluent, excipients and/or inert carrier.

In yet another aspect of the invention there is provided a pharmaceutical composition comprising the compound of formula I for use in the treatment of mGluR5 receptor mediated disorders, and for use in the treatment of neurological disorders, psychiatric disorders, gastrointestinal disorders and pain disorders.

In yet another aspect of the disclosure there is provided processes for the preparation of compounds of formula I.

A further aspect of the invention is the use of a compound according to formula I for the manufacture of a medicament for the treatment or prevention of functional gastrointestinal disorders, such as functional dyspepsia (FD). Yet another aspect of the invention is the use of a compound according to formula I for the manufacture of a medicament for the treatment or prevention of irritable bowel syndrome (IBS), such as constipation predominant IBS, diarrhea predominant IBS or alternating bowel movement predominant IBS.

These and other aspects of the present invention are described in greater detail herein below.

DETAILED DESCRIPTION OF THE INVENTION

The object of the present invention is to provide 5-substituted pyridines with surprisingly improved characteristics when compared to similarly substituted compounds, carrying other substituents at the 5-position of the pyridine ring.

Listed below are definitions of various terms used in the specification and claims to describe the present invention.

For the avoidance of doubt it is to be understood that where in this specification a group is qualified by 'hereinbefore defined', 'defined hereinbefore' or 'defined above' the said group encompasses the first occurring and broadest definition as well as each and all of the other definitions for that group.

For the avoidance of doubt it is to be understood that in this specification '$C_{1-6}$' means a carbon group having 1, 2, 3, 4, 5 or 6 carbon atoms.

In the case where a subscript is the integer 0 (zero) the group to which the subscript refers to indicates that the group is absent.

In this specification, unless stated otherwise, the term "alkyl" includes both straight and branched chain alkyl groups and may be, but are not limited to methyl, ethyl, n-propyl, i-propyl, n-butyl, i-butyl, s-butyl, t-butyl, n-pentyl, i-pentyl, t-pentyl, neo-pentyl, n-hexyl or i-hexyl, t-hexyl. The term $C_{1-3}$alkyl has 1 to 3 carbon atoms and may be methyl, ethyl, n-propyl or i-propyl.

In this specification, unless stated otherwise, the term "$OC_{0-6}$alkyl" includes both straight or branched alkoxy groups. $C_{0-3}$alkoxy may be, but is not limited to hydroxy, methoxy, ethoxy, n-propoxy or i-propoxy.

In this specification, unless stated otherwise, the term "halo"and "halogen" may be fluoro, chloro, bromo or iodo.

In this specification, unless stated otherwise, the term "alkylhalo" means an alkyl group as defined above, which is substituted with halo as described above.
The term "$C_{1-6}$alkylhalo" may include, but is not limited to fluoromethyl, difluoromethyl, trifluoromethyl, fluoroethyl, difluoroethyl or bromopropyl.
The term "$OC_{1-6}$alkylhalo" may include, but is not limited to fluoromethoxy, difluoromethoxy, trifluoromethoxy, fluoroethoxy or difluoroethoxy.

In one embodiment of the invention $X_1$ is N and $X_2$ is C or $X_1$ is C and $X_2$ is N.

The invention relates to compounds of formula I wherein $X_1$ is C and $X_2$ is N.
In another embodiment of the invention $X_1$ is N and $X_2$ is C.

In one embodiment of the invention Z is chloro. In one embodiment of the invention Z is fluoro.

In another embodiment of the invention $R^1$ and $R^2$ are selected from the group consisting of hydrogen, hydroxy,

halo,
$-C_{1-6}$alkylhalo, $-OC_{1-6}$alkylhalo, $-C_{1-6}$alkyl, $-OC_{0-6}$alkyl, $-C_{1-6}$alkylOR$^4$, $-OC_{2-6}$alkylOR$^4$, $-C_{0-6}$alkylcyano, $-C_{0-6}$alkylNR$^4$R$^5$ and $-OC_{2-6}$alkylNR$^4$R$^5$.
R$^4$ and R$^5$ are independently selected from the group consisting of hydrogen, hydroxy and $C_{1-3}$alkyl.
The invention relates to compounds of formula I wherein wherein R$^1$ and R$^2$ are selected from the group consisting of hydrogen, hydroxy, halo, $-C_{1-3}$alkylhalo, $-OC_{1-3}$alkylhalo, $-C_{1-3}$alkyl, $-OC_{0-3}$alkyl, $-C_{1-3}$allylOR$^4$, $-OC_2$-4alkylOR$^4$, $-C_{0-3}$alkylcyano and $C_{0-33}$alkylNR$^4$R,$^5$; and R$^4$ and R$^5$ are independently selected from hydrogen, methyl and ethyl.
In a further embodiment of the invention R$^1$ and R$^2$ are selected from the group consisting of fluoro, chloro, bromo, iodo, cyano, methoxymethyl, methoxy, difluoromethoxy, trifluoromethoxy, 2-methoxy-ethoxy, ethylamino and amine.
The invention relates to compounds of formula I wherein R$^1$ is fluoro, chloro, bromo, iodo, methoxymethyl, methoxy, difluoromethoxy, trifluoromethoxy, 2-methoxy-ethoxy, ethylamino or amine.
In another embodiment of the invention R$^1$ is methoxy, difluoromethoxy, trifluoromethoxy, 2-methoxy-ethoxy or ethylamino.
In yet a further embodiment of the invention R$^1$ is chloro, bromo, iodo or methoxymethyl. In yet another embodiment of the invention R$^1$ is fluoro.

In one embodiment of the invention R$^2$ is halo or $C_{0-6}$alkylcyano.
The invention relates to compounds of formula I wherein R$^2$ is fluoro or cyano.

The invention is also related to the following compounds;
3-fluoro-5-[5-(5-fluoropyridin-2-yl)-2H-tetrazol-2-yl]benzonitrile,
6-[2-(3-cyano-5-fluorophenyl)-2H-tetrazol-5-yl]nicotinonitrile,
3-[5-(5-chloropyridin-2-yl)-2H-tetrazol-2-yl]-5-fluorobenzonitrile,
3-[5-(5-fluoro-pyridin-2-yl)-tetrazol-2-yl]-5-methoxymethyl-benzonitrile,
3-fluoro-5-[2-(5-fluoropyridin-2-yl)-2H-tetrazol-5-yl]benzonitrile,
3-[2-(5-chloropyridin-2-yl)-2H-tetrazol-5-yl]-5-fluorobenzonitrile,
3-[5-(5-fluoropyridin-2-yl)-2H-tetrazol-2-yl]-5-(methoxymethyl)benzonitrile,
5-fluoro-2-[2-(3-fluoro-5-methoxyphenyl)-2H-tetrazol-5-yl]pyridine,
3-[5-(5-fluoro-pyridin-2-yl)-2H-tetrazol-2-yl]-5-methoxybenzonitrile,
3-[5-(5-fluoropyridin-2-yl)-2H-tetrazol-2-yl]-5-(trifluoromethoxy)benzonitrile,
3-(difluoromethoxy)-5-[5-(5-fluoropyridin-2-yl)-2H-tetrazol-2-yl]benzonitrile,
3-[5-(5-fluoropyridin-2-yl)-2H-tetrazol-2-yl]-5-(2-methoxyethoxy)benzonitrile,
3-(ethylamino)-5-[5-(5-fluoropyridin-2-yl)-2H-tetrazol-2-yl]benzonitrile,
3-ammo-5-[5-(5-fluoropyridin-2-yl)-2H-tetrazol-2-yl]benzonitrile,
3-[5-(5-fluoropyridin-2-yl)-2H-tetrazol-2-yl]-5-iodobenzonitrile, and
or salts, solvates or solvated salts thereof.

A suitable pharmaceutically acceptable salt of the compounds of the invention is, for example, an acid-addition salt, for example an inorganic or organic acid. In addition, a suitable pharmaceutically acceptable salt of the compounds of the invention is an alkali metal salt, an alkaline earth metal salt or a salt with an organic base.
Other pharmaceutically acceptable salts and methods of preparing these salts may be found in, for example, Remington's Pharmaceutical Sciences (18$^{th}$ Edition, Mack Publishing Co.) 1990.

Some compounds of formula I may have chiral centres and/or geometric isomeric centres (E- and Z- isomers), and it is to be understood that the invention encompasses all such optical, diastereoisomeric and geometric isomers.

The invention also relates to any and all tautomeric forms of the compounds of formula I.

## Pharmaceutical composition

According to one aspect of the present invention there is provided a pharmaceutical composition comprising as active ingredient a therapeutically effective amount of the compound of formula I, or salts, solvates or solvated salts thereof, in association with one or more pharmaceutically acceptable diluent, excipients and/or inert carrier.

The composition may be in a form suitable for oral administration, for example as a tablet, pill; syrup, powder, granule or capsule, for parenteral injection (including intravenous, subcutaneous, intramuscular, intravascular or infusion) as a sterile solution, suspension or emulsion, for topical administration e.g. as an ointment, patch or cream or for rectal administration e.g. as a suppository.
In general the above compositions may be prepared in a conventional manner using one or more conventional excipients, pharmaceutical acceptable diluents and/or inert carriers.

Suitable daily doses of the compounds of formula I in the treatment of a mammal, including man are approximately 0.01 to 250 mg/kg bodyweight at peroral administration and about 0.001 to 250 mg/kg bodyweight at parenteral administration.
The typical daily dose of the active ingredients varies within a wide range and will depend on various factors such as

the relevant indication, severity of the illness being treated, the route of administration, the age, weight and sex of the patient and the particular compound being used, and may be determined by a physician.

**Medical use**

It has been found that the compounds according to the present invention, salts, solvates or solvated salts thereof, exhibit a high degree of potency and selectivity for individual metabotropic glutamate receptor (mGluR) subtypes. Accordingly, the compounds of the present invention are expected to be useful in the treatment of conditions associated with excitatory activation of an mGluR5 receptor and for inhibiting neuronal damage caused by excitatory activation of an mGluR5 receptor. The compounds may be used to produce an inhibitory effect of mGluR5, in mammals, including man. The mGluR5 receptor is highly expressed in the central and peripheral nervous system and in other tissues. Thus, it is expected that the compounds of the invention are well suited for the treatment of mGluR5 receptor-mediated disorders such as acute and chronic neurological and psychiatric disorders, gastrointestinal disorders, and chronic and acute pain disorders.

The invention relates to compounds of formula I as defined hereinbefore, for use in therapy.

The invention relates to compounds of formula I as defined hereinbefore, for use in treatment of mGluR5 receptor-mediated disorders.

The invention relates to compounds of formula I as defined hereinbefore, for use in treatment of Alzheimer's disease senile dementia, AIDS-induced dementia, Parkinson's disease, amylotropic lateral sclerosis, Huntington's Chorea, migraine, epilepsy, schizophrenia, depression, anxiety, acute anxiety, ophthalmological disorders such as retinopathies, diabetic retinopathies, glaucoma, auditory neuropathic disorders such as tinnitus, chemotherapy induced neuropathies, post-herpetic neuralgia and trigeminal neuralgia, tolerance, dependency, Fragile X, autism, mental retardation, schizophrenia and Down's Syndrome. The invention relates to compounds of formula I as defined hereinbefore, for use in treatment of pain related to migraine, inflammatory pain, neuropathic pain disorders such as diabetic neuropathies, arthritis and rheumatitiod diseases, low back pain, post-operative pain and pain associated with various conditions including angina, renal or billiary colic, menstruation, migraine and gout.

The invention relates to compounds of formula I as defined hereinbefore, for use in treatment of stroke, head trauma, anoxic and ischemic injuries, hypoglycemia, cardiovascular diseases and epilepsy.

The present invention relates also to the use of a compound of formula I as defined hereinbefore, in the manufacture of a medicament for the treatment of mGluR5 receptor mediated disorders and any disorder listed above.

One embodiment of the invention relates to the use of a compound according to formula I in the treatment of gastrointestinal disorders.

Another embodiment of the invention relates to the use of a compound according to formula I, for the manufacture of a medicament for the inhibition of transient lower esophageal sphincter relaxations, for the treatment of GERD, for the prevention of reflux, for the treatment regurgitation, treatment of asthma, treatment of laryngitis, treatment of lung disease and for the management of failure to thrive.

The invention also provides a method of treatment of mGluR5 receptor mediated disorders and any disorder listed above, in a patient suffering from, or at risk of, said condition, which comprises administering to the patient an effective amount of a compound of formula I, as hereinbefore defined.

The dose required for the therapeutic or preventive treatment of a particular disorder will necessarily be varied depending on the host treated, the route of administration and the severity of the illness being treated.

In the context of the present specification, the term "therapy" and "treatment" includes prevention and/or prophylaxis, unless there are specific indications to the contrary. The terms "therapeutic" and "therapeutically" should be construed accordingly.

In this specification, unless stated otherwise, the term "antagonist" and "inhibitor" shall mean a compound that by any means, partly or completely, blocks the transduction pathway leading to the production of a response by the ligand.

The term "disorder", unless stated otherwise, means any condition and disease associated with metabotropic glutamate receptor activity.

**Non- Medical use**

In addition to their use in therapeutic medicine, the compounds of formula I, or salts, solvates or solvated salts thereof, are also useful as pharmacological tools in the development and standardisation of *in vitro* and *in vivo* test systems for the evaluation of the effects of inhibitors of mGluR related activity in laboratory animals such as cats, dogs, rabbits, monkeys, rats and mice, as part of the search for new therapeutics agents.

*Methods of Preparation*

Another aspect of the present invention provides processes for preparing compounds of formula I, or salts, solvates or solvated salts thereof. Processes for the preparation of the compounds in the present invention are described herein.

Throughout the following description of such processes it is to be understood that, where appropriate, suitable protecting groups will be added to, and subsequently removed from, the various reactants and intermediates in a manner that will be readily understood by one skilled in the art of organic synthesis. Conventional procedures for using such protecting groups as well as examples of suitable protecting groups are described, for example, in "Protective Groups in Organic Synthesis", T.W. Green, P.G.M. Wuts, Wiley-Interscience, New York, (1999). It is also to be understood that a transformation of a group or substituent into another group or substituent by chemical manipulation can be conducted on any intermediate or final product on the synthetic path toward the final product, in which the possible type of transformation is limited only by inherent incompatibility of other functionalities carried by the molecule at that stage to the conditions or reagents employed in the transformation. Such inherent incompatibilities, and ways to circumvent them by carrying out appropriate transformations and synthetic steps in a suitable order, will be readily understood to the one skilled in the art of organic synthesis. Examples of transformations are given below, and it is to be understood that the described transformations are not limited only to the generic groups or substituents for which the transformations are exemplified. References and descriptions on other suitable transformations are given in "Comprehensive Organic Transformations - A Guide to Functional Group Preparations" R. C. Larock, VHC Publishers, Inc. (1989). References and descriptions of other suitable reactions are described in textbooks of organic chemistry, for example, "Advanced Organic Chemistry", March, 4th ed. McGraw Hill (1992) or, "Organic Synthesis", Smith, McGraw Hill, (1994). Techniques for purification of intermediates and final products include for example, straight and reversed phase chromatography on column or rotating plate, recrystallisation, distillation and liquid-liquid
or solid-liquid extraction, which will be readily understood by the one skilled in the art.

The definitions of substituents and groups are as in formula I except where defined differently. The term "room temperature" and "ambient temperature" shall mean, unless otherwise specified, a temperature between 16 and 25 °C.

Compounds of formula II and III are either useful as intermediates in the preparation of compounds of formula I, or may represent compounds of formula I (scheme 1).

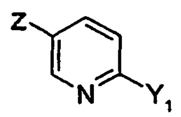

(II)

$Y_1$=

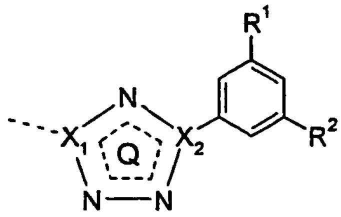

or CHO, $NH_2$, CN or $B(OH)_2$

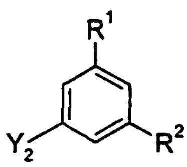

(III)

$Y_2$=

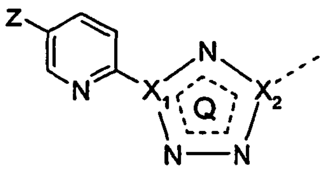

or CHO, $NH_2$, CN or $B(OH)_2$

Z=Br, I and as defined in formula I

Scheme 1

Compounds of formula II and III are either commercially available, or can be prepared from either commercially available, or in the literature described compounds. For example, compounds in which one or more of Z, $Y_1$, $Y_2$, $R^1$ or $R^2$ does not correspond to the definitions of formula II or III, can by used for the preparation of compounds of formula II and III by transformation or introduction of substituents or groups.

Examples are described below.

1) Preparation of compounds of formula II or III in which $Y_1$ and $Y_2$ respectively is CHO. 1a) From the corresponding nitriles by reduction using for example DIBAL in methylenechloride or toluene at-78 to 0 °C, or $SnCl_2$ under aqueous

acidic conditions.

1b) From the corresponding bromides or iodides by palladium catalysed carbonylation using for example Pd $(PPh_3)_4$ as catalyst under 1-10 atm CO in the presence of stochiometric amounts of e.g. $Ph_3SnH$ in THF or toluene at a temperature of room temperature to 150 °C (J. Am. Chem. Soc. 1983, 7175-7176).
Alternatively, from the corresponding chlorides, bromides or iodides by initial conversion into a nucleophilic organolithium, ate-complex or Grignard reagent by treatment with for example *n*BuLi, $n\mathrm{Bu_3MgLi}$ or Mg, followed by trapping with DMF or another formylating compound (Tetrahedron Letters 2000, 4335-4338 or J. Org. Chem. 2001, 6775-6786).
1c) By electrophilic aromatic formylation of the corresponding benzene derivative in which $Y_2$ is H using for example $POCl_3$ and DMF, or $CHCl_2OCH_3$ and $TiCl_4$, as formylation reagents.
1d) From the corresponding methyl derivatives by oxidation using Cr(VI)-compounds, such as $CrO_3$ in HOAc, or other suitable oxidants.

2) Preparation of compounds of formula II or III in which $Y_1$ and $Y_2$ respectively is $NH_2$. 2a) By reduction of the corresponding nitro derivatives by hydrogenation at 1-10 atm $H_2$ in the presence of a catalyst such as Pd on carbon, $PtO_2$ or Raney-Ni, or by treatment with reducing agents such as Fe-powder in mixtures of MeOH and aqueous $NH_4Cl$, or $SnCl_2$ in aqueous HCl.

2b) From the corresponding carboxylic acids via a Curtius rearrangement initiated by treatment with $N_3PO(OPh)_2$ and triethylamine in boiling *t*BuOH, followed by acidic removal of the BOC-group from the resulting N-BOC amine using for example HCl in EtOAc.
2c) From the corresponding chlorides, bromides, iodides or triflates by palladium catalysed amination employing benzophenone imine as a masked ammonia equivalent. As catalysts suitable sources of palladium, such as $Pd(OAc)_2$, $Pd(dba)_2$ or $Pd_2(dba)_3$, together with additives serving as ligands, such as BNAP, DPPF or 1,3-bis (2,6-diisopropylphenyl)imidazolium chloride, are used in combination with stochiometric amount of bases, such as $Cs_2CO_3$ or *t*BuONa, in solvents, such as THF, toluene or dioxane, at a temperature from room temperature to 120 °C (Tetrahedron Letters 1997, 6367-6370; J. Org. Chem. 2001, 7729-7737 or J. Org. Chem. 2001, 6775-6786). The formed imine is then cleaved by for example acidic hydrolysis or hydrogenolysis to free the amine.

3) Preparation of compounds of formula II or III in which Z, $R^1$ or $R^2$ or $Y_1$ and $Y_2$ respectively is CN.

3a) From the corresponding chlorides, bromides or iodides by displacement using various sources of cyanide, such as CuCN or KCN, in solvents, such as pyridine or DMF, at temperatures from room temperature to 200 °C, or via palladium catalysed protocols using catalytic amounts of for example $Pd(PPh_3)_4$ together with stochiometric amounts of for example $Zn(CN)_2$, or NaCN with added catalytic amounts of CuI, in solvents, such as DMF, acetonitrile or propionitrile, with conventional heating at a temperature of 50 to 150 °C, or by microwave irradiation from at a temperature of 100 to 200 °C (J. Org. Chem. 1998, 8224-8228 or J. Org. Chem. 2000, 7984-7989).
3b) From the corresponding amines via diazotation, using for example $HNO_2$ or alkyl nitrites, such as tbutyl nitrite, in a suitable solvent such as water, alcohols or acetonitrile, followed by reaction with aqueous mixtures of CuCN and KCN at a temperature of room temperature to 100 °C.
3c) From the corresponding aldehydes via formation of the oxime by condensation with hydroxylamine, followed by dehydration using for example $Ac_2O$.

4) Preparation of compounds of formula II or III in which Z, $R^1$ or $R^2$ or $Y_1$ and $Y_2$ respectively is F.

4a) From the corresponding chloride, bromide, iodide or nitro compound by displacement with nucleophilic fluorides such as lithium fluoride, tetrabutylammonium fluoride or tetramethylammonium hydrogendifluoride in for example DMF, DMA or the like as solvent at a temperature of room temperature to 150 °C.
4b) From the corresponding amine by the Schiemann reaction, eg via diazotation to the corresponding diazonium fluoroborate followed by thermal decomposition at a temperature of 30 to 150 °C of this. The Schiemann reaction conditions were found not to work in the case of compounds of Formula II wherein $Y_1$ is CN. For such compounds an alternative process was developed using 70% HF-pyridine to generate the diazonium species followed by *in situ* decomposition at elevated temperatures, for example at 80°C. This process was used to generate the desired compound of Formula II in which Z is F and $Y_1$ is CN, which was used as a key intermediate with reduction to the aldehyde as described above in section 1a) followed by subsequent conversion to the hydrazone

and cyclization onto the diazonium salt to give cpds of formula I. This process provides a high yield (>90%), and pure product without chromatography. The intermediate formed is very versatile and can be used to make many desired final products.

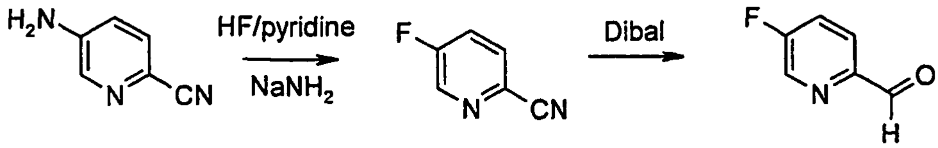


Scheme 2

5) Preparation of compounds of formula II or III in which $Y_1$ and $Y_2$ respectively is $B(OH)_2$.

5a) From the corresponding chlorides, bromides, iodides or triflates through palladium catalysed coupling with commercially available bis(pinacolato)diboran, using for example $PdCl_2$(DPPF), or $Pd(dba)_2$ with added tricyclohexylphosphine, as catalysts, together with stochiometric amounts of KOAc in solvents such as DMSO, DMF, DMA or dioxan at a temperature of room temperature to150 °C followed by acidic hydrolysis (Tetrahedron 2001, 9813-9816).

5b) From the corresponding chlorides, bromides or iodides by initial conversion into a nucleophilic organolithium, ate-complex or Grignard reagent by treatment with for example *n*BuLi, *n*$Bu_3$MgLi or Mg, followed by trapping with for example triisopropyl borate or the like and subsequent acidic hydrolysis.

Compounds of formula II in which $Y_1$ is CHO, may for example also be prepared from the corresponding methyl compounds according to the procedure of for example French et al (J. Med. Chem. 1970, 1124-1130) by initial N-oxidation of the pyridine, using for example AcOOH or mCPBA as oxidant at a temperature of 0 °C to room temperature in for example chloroform or methylene chloride as solvent, followed by rearrangement of the pyridine N-oxide in $Ac_2O$ as solvent at a temperature of 80 to 120 °C, and acidic or basic hydrolysis of the resulting 2-pyridylmethanol acetate to yield the corresponding 2-pyridylmethanol derivative, which is then oxidized with for example activated $MnO_2$ in chloroform or similar solvents at a temperature of room temperature to 80 °C (Scheme 3).

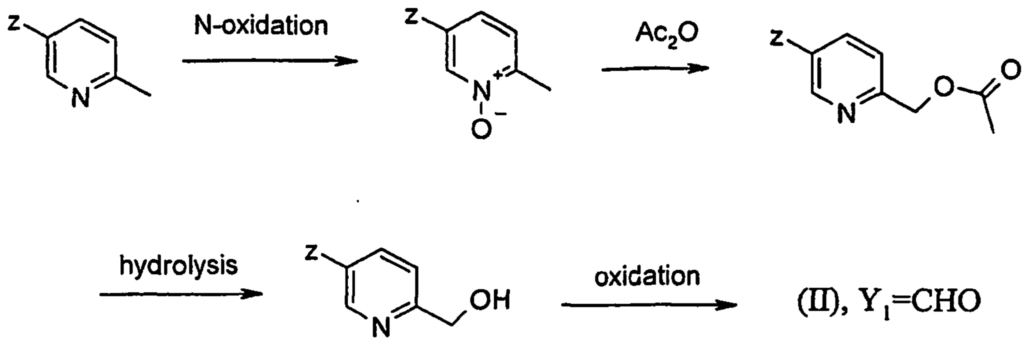


Scheme 3

Compounds of formula I may be prepared by reacting arylsulphonylhydrazides of formula IV or VII with diazonium compounds of formula V and VI respectively, in for example pyridine, alcohols or DMF as solvent at a temperature of 0 to 100 °C as described in scheme 4 (Helv.Chim.Acta 1985, 1283-1300, WO 03/029210 and WO 03/077918). Diazonium compounds are prepared from the corresponding amines with for example $HNO_2$ or alkyl nitrites, such as tbutyl nitrite, in a suitable solvent such as water, alcohols or acetonitrile. The diazonium salts thus formed may be used "in situ" or may be precipitated as the tetrafluoroborate salt when $HBF_4$ is used as the acid and fluorine source. Arylsulphonylhydrazides, such as p-tolylsulphonylhydrazides, are prepared through condensation between aldehydes of formula II or III ($Y_1$ and $Y_2$ respectively is CHO) with arylsulphonylhydrazines in for example methanol, ethanol, DMF or dialkylethers

at a temperature of 0 to 100 °C, alternatively without solvent under microwave irradiation (J. Med. Chem.1980, 631-634 and Monatshefte fuer Chemie 2001, 403-406).

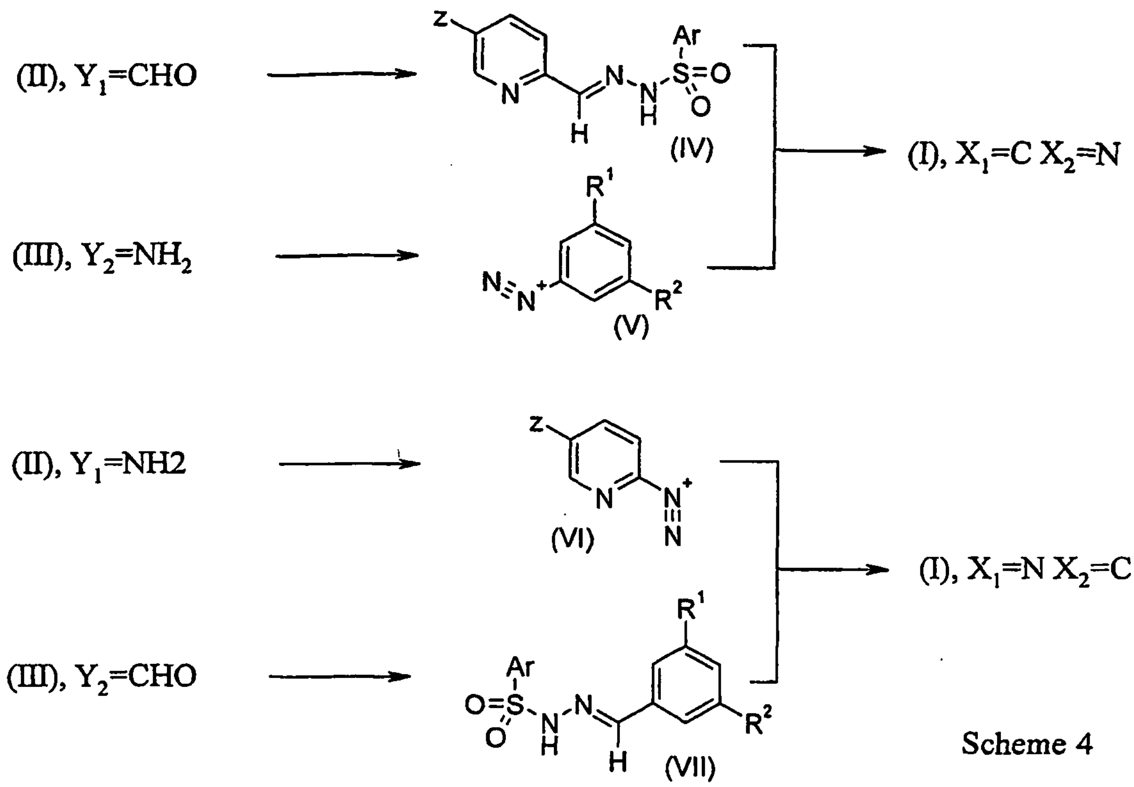


Scheme 4

Compounds of formula I may also be prepared as described in scheme 5 by N2-arylation of tetrazoles of formula VIII and IX using for example boronic acids of formula II and III ($Y_1$ and $Y_2$ respectively is $B(OH)_2$), or the corresponding iodonium salts of formula IX and XI, or the corresponding triaryl bismuth diacetates of formula X and XII, as arylating agents mediated by transition metals (Tetrahedron Letters 2002, 6221-6223 and Tetrahedron Letters 1998, 2941-2944 and Tetrahedron Lett 1999,2747-2748). With boronic acids, stochiometric amounts of Cu(II)acetate and pyridine are used in solvents such as methylenechloride, DMF, dioxane or THF at a temperature of room temperature to 100 °C. With iodonium salts, catalytic amounts of Pd(II)-compounds, such as $Pd(dba)_2$ or $Pd(OAc)_2$, together with catalytic amounts of Cu(II)-carboxylates, such as Cu(II)-phenylcyclopropylcarboxylate, and bidentate ligands, such as BINAP or DPPF, are used in solvents such as tBuOH at a temperature of 50 to 100 °C. With triaryl bismuth diacetates, catalytic amounts of cupric acetate may be employed in the presence of N,N,N',N'-tetramethylguanidine in a suitable solvent such as THF with heating at a temperature of 40-60°C. Iodonium salts of formula IX and XI may be obtained from, for example, the respective boronic acids of formula II and III ($Y_1$ and $Y_2$ respectively is $B(OH)_2$) by treatment with hypervalent iodine substituted aromatics, such as hydroxyl(tosyloxy)iodobenzene or $PhI(OAc)_2$x2TfOH, in dichloromethane or the like (Tetrahedron Letters 2000, 5393-5396). Triarylbismuth diacetates are readily available from aryl magnesium bromides with bismuth trichloride in a suitable solvent such as refluxing THF to give the triarylbismuthane, which is then oxidized to the diacetate using an oxidizing agent such as sodium perborate in acetic acid. (Synth. Commun. 1996, 4569-75) The tetrazoles of formula VIII and XIII are obtained from, for example, the respective nitriles of formula II and III ($Y_1$ and $Y_2$ respectively is CN) by treatment with an azide, such as $NaN_3$, $LiN_3$, trialkylyltinazide or trimethylsilylazide, preferrably with a catalyst such as dibutyltin oxide or $ZnBr_2$, in solvents such as DMF, water or toluene at a temperature of 80 to 200 °C by conventional heating or microwave irradiation (J. Org. Chem. 2001, 7945-7950; J. Org. Chem. 2000, 7984-7989 or J. Org. Chem. 1993, 4139-4141).

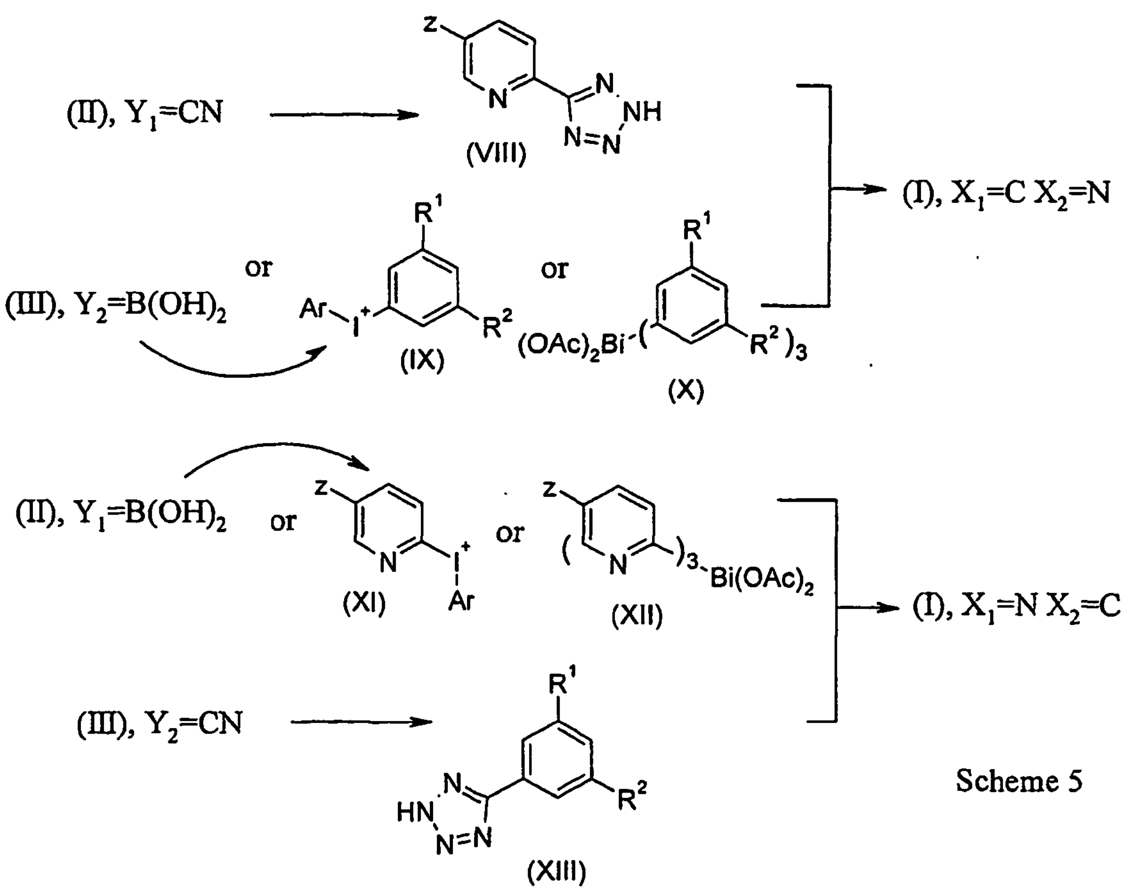


Scheme 5

## Examples

The invention will now be illustrated by the following non-limiting examples.

### General methods

All starting materials are commercially available or earlier described in the literature.
The $^1$H and $^{13}$C NMR spectra were recorded either on Bruker 300, Bruker DPX400 or Varian +400 spectrometers operating at 300, 400 and 400 MHz for $^1$H NMR respectively, using TMS or the residual solvent signal as reference, in deuterated chloroform as solvent unless otherwise indicated. All reported chemical shifts are in ppm on the delta-scale, and the fine splitting of the signals as appearing in the recordings (s: singlet, br s: broad singlet, d: doublet, t: triplet, q: quartet, m: multiplet).
Analytical in line liquid chromatography separations followed by mass spectra detections, were recorded on a Waters LCMS consisting of an Alliance 2795 (LC) and a ZQ single quadropole mass spectrometer. The mass spectrometer was equipped with an electrospray ion source operated in a positive and/or negative ion mode. The ion spray voltage was $\pm$3 kV and the mass spectrometer was scanned from m/z 100-700 at a scan time of 0.8 s. To the column, X-Terra MS, Waters, C8, 2.1, x 50mm, 3.5 $\mu$m, was applied a linear gradient from 5 % to 100% acetonitrile in 10 mM ammonium acetate (aq.), or in 0.1% TFA (aq.). Preparative reversed phase chromatography was run on a Gilson autopreparative HPLC with a diode array detector using an XTerra MS C8, 19x300mm, 7$\mu$m as column.
MS-triggered preparative reversed phase chromatography was run on a Waters autopurification LC-MS system with a diode array detector and a ZQ mass detector using an XTerra MS C8, 19x100 mm, 5 $\mu$m as column.
Purification by a chromatotron was performed on rotating silica gel / gypsum (Merck, 60 PF-254 with calcium sulphate) coated glass sheets, with coating layer of 1, 2, or 4 mm using a TC Research 7924T chromatotron.
Purification of products were also done using Chem Elut Extraction Columns (Varian, cat #1219-8002), Mega BE-SI (Bond Elut Silica) SPE Columns (Varian, cat # 12256018; 12256026; 12256034), or by flash chromatography in silica-filled glass columns. Microwave heating was performed in a Smith Synthesizer Single-mode microwave cavity producing continuous irradiation at 2450 MHz (Personal Chemistry AB, Uppsala, Sweden).

**Example 1: 3-Fluoro-5-[5-(5-fluoropyridin-2-yl)-2*H*-tetrazol-2-yl]benzonitrile**

A diazonium chloride solution was prepared from 3-amino-5-fluorobenzonitrile (0.10 g) in ethanol (2 mL), sodium nitrite (0.06 g) in water (1 mL) and 10 % aqueous HCl solution (2 mL) at 5 °C. This solution was added dropwise, with stirring, to a solution of *N'*-[(1*E*)-(5-fluoropyridin-2-yl)methylene]-4-methylbenzenesulfonohydrazide (0.15 g) in pyridine (5 mL) over 10 minutes, such that the temperature remained below 5 °C. The reaction mixture was stirred for 2 h, then concentrated, and the resultant products were partitioned between $CH_2Cl_2$ (100 mL) and water (50 mL). The organic phase was washed with water (50 mL), and brine (50 mL), then dried ($Na_2SO_4$), filtered and concentrated. The residue was recrystallized from EtOAc to give 40 mg of the title compound as a yellow solid.
$^1$H NMR: 7.56 (m, 1H), 7.67 (m, 1H), 8.3 3 (m, 1H), 8.42 (m, 1H), 8.47 (m, 1H), 8.73 (m, 1H).

**3-amino-5-fluorobenzonitrile**

To a slurry of 3-fluoro-5-nitro-benzonitrile (J.Antibiot. 1994, 1456-1465) (0.20 g) in 2-propanol (4 mL) and conc. aq. HCl (2 mL) was added tin(II)chloride dihydrate (1.1 g). The mixture was refluxed for 1 h, then cooled and concentrated. The residue was suspended in water and basified with 1 N NaOH solution. The product was taken up in $CH_2Cl_2$ (125 mL), washed with brine (50 mL), dried ($Na_2SO_4$), filtered and concentrated to give 0.16 g of the title compound as a white solid.
$^1$H NMR: 4.03 (br s, 2H), 6.58 (m, 1H), 6.71 - 6.75 (2H).

***N*-[(1*E*)-(5-Fluoropyridin-2-yl)methylene]-4-methylbenzenesulfonohydrazide**

5-Fluoropyridine-2-carbaldehyde (0.18 g) was added to an ethanol (10 mL) solution of p-toluenesulfonyl hydrazide (0.26 g). A catalytic amount of acetic acid was added, and the reaction was stirred at room temperature for 1.5 h, then cooled to 0 °C and filtered. The precipitate was dried under high vacuum to afford 0.23 g of the title compound as a white solid.
$^1$H NMR: 2.44 (s, 3H), 7.33 (m, 2H), 7.44 (m, 1H), 7.82 (d, 1H), 7.88 (m, 1H), 7.96 (m, 1H), 8.17 (br s, 1H), 8.41 (m, 1H).

**5-Fluoro-pyridine-2-carbaldehyde p. 3600-35**

DIBAL (1 M toluene solution, 8.2 mL, 8.2 mmol) was added dropwise to a cold (-78 °C) solution of 5-fluoropyridine-2-carbonitrile (1.0g, 8.2 mmol) in $CH_2Cl_2$ (50 mL) and the resulting misture was stirred at -78°C for 3 h, with an additional 0.25 equivalents of DIBAL added after 0.5 and 1 h. The reaction was quenched with 1 N HCl (20 mL) and stirred for 2.5 h. The aqueous phase was basified with solid $NaHCO_3$ and the product was extracted with $CH_2Cl_2$. (An emulsion formed that required filtration before extraction could be performed). The combined organics were washed with brine and dried ($Na_2SO_4$) and filtered. Purification was performed by flash column chromatography over silica gel, eluting with 2 % EtbAc/hexanes to yield the product (200 mg, 20 %, colourless solid which melts just above room temperature and was volatile under high vacuum resulting in a lower yield than expected). **$^1$H NMR ($CDCl_3$, 300 MHz):** $\delta$ = 7.59 (ddd, J = 8, 2, 1 Hz, 1H), 8.04 (ddd, J = 8, 5, 0.5 Hz, 1H), 8.64 (d, J = 2 Hz, 1H), 10.00 (apparent dd, J = 1, 1 Hz, 1H). Alternatively this compound can be made by the method described in J. Med Chem. 1970, 1124-1130.

**5-Fluoro-pyridine-2-carbonitrile**

Sodium nitrite (8.7g, 126 mmol) was added portionwise to an ice-salt cooled solution of 5-amino-pyridine-2-carbonitrile (10.03g, 84.2 mmol) in 70% hydrogen fluoride - pyridine (Aldrich, 100g, 3.5 mol HF) (note: the reaction was carried out in the supplied bottle). The resulting dark red solution was stirred for 45 min in the ice-salt bath, then the bath was removed and the mixture stirred at ambient temperature for 30 min, followed by heating at 80°C for 1.5h. The reaction mixture was quenched by pouring onto ice/water mixture (~400g) in a separatory funnel and extracted with dichloromethane (6x150mL), dried (MgSO4), filtered and evaporated the solvent *in vacuo.* The crude product (10.08g, 98%, orange solid) was sufficiently pure for use without further purification. **$^1$H NMR ($CDCl_3$, 300 MHz):** $\delta$ = 8.61 (d, 1H), 7.78 (m, 1H), 7.58 (m, 1H). GC-MS M+122.

**Example 2: 3-[5-(5-Chloropyridin-2-yl)-2*H*-tetrazol-2-yl]-5-fluorobenzonitrile**

The title compound was prepared analogously to 3-fluoro-5-[5-(5-fluoropyridin-2-yl)-2*H*-tetrazol-2-yl]benzonitrile from 3-amino-5-fluoro-benzonitrile (0.14 g) and *N'*-[(1*E*)-(5-chloropyridin-2-yl)methylene]-4-methylbenzenesulfonohydrazide (0.22 g) to afford 67 mg of the title compound as an orange solid.
$^1$H NMR: 7.56 (m, 1H), 7.95 (m, 1H), 8.31 - 8.37 (2H), 8.47 (m, 1H), 8.83 (m, 1H)

**N'-[(1E)-(5-Chloropyridin-2-yl)methylene]-4-methylbenzenesulfonohydrazide**

The title compound was prepared analogously to N'-[(1E)-(5-fluoropyridin-2-yl)methylene]-4-methylbenzenesulfonohydrazide from 5-chloropyridine-2-carbaldehyde [J. Med. Chem. 1970, 1124-30] (1.0 g) and p-toluenesulfonyl hydrazide (1.0 g) to afford 0.54 g of the title compound as a white solid.
$^1$H NMR: 2.44 (s, 3H), 7.34 (m, 2H), 7.69(m, 1H), 7.80 (d, 1H), 7.86 - 7.91 (3H), 8.16 (br s, 1H), 8.51 (m, 1H).

**Example 3: 6-[2-(3-Cyano-5-fluorophenyl)-2H-tetrazol-5-yl]nicotinonitrile**

3-[5-(5-bromopyhdin-2-yl)-2H-tetrazol-2-yl]-5-fluorobenzonitrile (WO 03/029210) (0.03 g) was dissolved in DMF (3 ml), and argon was bubbled through for 15 minutes. Zinc cyanide (0.01 g) and $Pd(PPh_3)_4$ (0.03 g) were added, and the reaction stirred at 80 °C for 16 h. The reaction mixture was diluted with $CH_2Cl_2$ (20 mL) and washed with $NH_4Cl$ (10 mL) and brine (10 mL), then dried ($Na_2SO_4$), filtered and concentrated to give a yellow solid that was purified by recrystallization from EtOAc:hexanes to afford 7 mg of the title compound as a white solid.
$^1$H NMR: 7.59 (m, 1H), 8.25 (m, 1H), 8.34 (m, 1H), 8.47 (m, 1H), 8.53 (m, 1H), 9.13 (m, 1H).

**Example 4: 3-[5-(5-fluoropyridin-2-yl)-2H-tetrazol-2-yl]-5-(methoxymethyl)benzonitrile**

A diazonium chloride solution was prepared from 3-amino-5-methoxymethyl-benzonitrile (45 mg, 0.28 mmol) in ethanol (1 mL), sodium nitrite (21 mg, 0.30 mmol) in water (0.5 mL) and 10 % aqueous HCl solution (1 mL) at 5 °C. This solution was added dropwise, with stirring, to a solution of N'-[(1E)-(5-fluoropyridin-2-yl)methylene]-4-methylbenzene-sulfonohydrazide (81 mg. 0.28 mmol) in pyridine (2.5 mL) over 10 minutes, such that the temperature remained below 5°C. The reaction mixture was stirred at 0°C for 0.5 h and at r.t. for 1.5 h, then concentrated, and the resultant products were partitioned between EtOAc (10 mL) and brine (10 mL). The water phase was washed with EtOAc (10 mL) and the combined organic phase dried ($Na_2SO_4$), filtered and concentrated. The residue was purified by flash column chromatography over silica gel, eluting with EtOAc/toluene (1:3) followed by recrystallized from EtOAc to give 14 mg of the title compound as a yellow solid.
$^1$H NMR: 3.51 (s, 3 H), 4.62 (s, 2 H), 7.65 (m, 1 H), 7.81 (s, 1H), 8.41 (m, 1H), 8.52 (s, 2 H), 8.72 (br. s., 1 H)

**3-aming-5-methoxymethyl-benzonitrile**

TFA (0.56 mL, 7.2 mmol) was added to (3-Cyano-5-methoxymethyl-phenyl)-carbamic acid tert-butyl ester (189 mg, 0.72 mmol) in $CH_2Cl_2$ (5 mL). The mixture was stirred at r.t. for 1.5 h and then concentrated. The residue was portion between $CHCl_3$ (10 mL) and $NaHCO_3$ (10 mL). The water phase was washed with $CHCl_3$ (10 mL) and the combined organic phase dried ($MgSO_4$), filtered and concentrated. The residue was purified by flash column chromatography over silica gel, eluting with $CHCl_3$/MeOH (98:2) to give 82 mg of the title compound.
$^1$H NMR: 3.38 (s, 3 H), 3.90 (br. s., 2 H), 4.35 (s, 2 H), 6.80 (s, 1 H), 6.84 (s, 1 H), 6.95 (s, 1H)
$^{13}$C NMR: 58.8, 79.9, 113.2, 117.1, 118.4, 119.6, 121.0, 141.4, 147.7

**(3-Cyano-5-methoxymethyl-phenyl)-carbamic acid tert-butyl ester**

$Et_3N$ (0.61 mL, 4.4 mmol) and diphenylphosphoryl azide (0.52 mL, 2.4 mmol) was added to 3-Cyano-5-methoxymethyl-benzoic acid (382 mg, 2 mmol) under an argon-atmosphere. The mixture was refluxed for 4.5 h, concentrated, and the resultant product was partitioned between EtOAc (50 mL) and brine (25 mL). The organic phase was washed with brine (25 mL) and the combined organic phase was dried ($MgSO_4$) filtered and concentrated. The residue was purified by flash column chromatography over silica gel, eluting with $CHCl_3$ to $CHCl_3$/MeOH (95:5) to give 190 mg of the title compound.
$^1$H NMR: 49 (s, 9 H), 3.37 (s, 3 H), 4.40 (s, 2 H), 7.19 (s, 1 H), 7.24 (s, 1 H), 7.52 (s, 1 H), 7.70 (s, 1 H)
$^{13}$C NMR: 28.6, 58.8, 73.7, 81.7, 113.2, 119.0, 120.9, 121.7, 125.3, 140.1, 141.16, 153.0

**3-Cyano-5-methoxymethyl-benzoic acid**

0.5 M LiOH (aq) (9.7 mL, 4.9 mmol) was added to 3-Cyano-5-methoxymethyl-benzoic acid methyl ester (1g, 4.9 mmol) and the mixture was stirred at r.t. for 2.5 h. 1M HCl was added until pH=2 followed by $H_2O$ (25 mL) and $CHCl_3$ (50 mL). The mixture was extracted and the phases were separated. The water phase was washed with $CHCl_3$ (10 mL) and the combined organic phases were dried ($MgSO_4$) filtered and concentrated to give 0.93g of the title compound.
$^1$H NMR: 3.47 (s, 3 H), 4.56 (s, 2 H), 7.87 (m, 1 H), 8.27 (m, 2 H), 10.64 (br. s., 1 H)
$^{13}$C NMR: 58.6, 72.7, 113.1, 117.6, 132.7, 135.17, 140.8, 169.3

**3-Cyano-5-methoxymethyl-benzoic acid methyl ester**

Trifluoroacetic anhydride (16.8 mL, 119 mmol) followed by pyridine (16.9 mL, 210 mmol) was added to 5-methoxymethyl-isophthalamic acid methyl ester (21 g, 95 mmol) in $CH_2Cl_2$ (500 mL) at 0 °C. The mixture was stirred at 0 °C for 20 minutes and at r.t. over night. The solvent was evaporated to give 19g of the title compound.
$^1$H NMR: 3.45 (s, 3 H), 3.97 (s, 3 H), 4.53 (s, 2 H), 7.84 (s, 1 H), 8.23 (d, 2 H)
$^{13}$C NMR: 52.7, 58.7, 72.9, 113.5, 132.3, 132.3, 134.5, 140.7, 165.1

**5-Methoxymethyl-isophthalamic acid methyl ester**

5-Methoxymethyl-isophthalic acid monomethyl ester (24.3 g, 108 mmol) and $SOCl_2$ (60 mL, 760 mmol) was heated to 60 °C for 2 h. The mixture was cooled and the excess $SOCl_2$ was evaporated. 2M $NH_3$ in MeOH (220 mL, 430 mmol) was added to the residue in THF (200 mL) at 0 °C and was stirred at 0 °C for 1h. The mixture was filtered and the solvent was removed in vacuum to give 24.2 g of the title compound as a white solid.
$^1$H NMR: 3.44 (s, 3 H), 3.95 (s, 3 H), 4.55 (s, 2 H), 6.22 (br. d., 2 H), 8.06 (s, 1 H), 8.17 (s, 1 H), 8.38 (s, 1 H)
13$^C$ NMR: 52.4, 58.5, 73.6, 127.4, 130.8, 130.9, 131.8, 133.8, 139.7, 166.2, 168.3

**5-Methoxymethyl-isophthalic acid monomethyl ester**

1 M NaOH (aq) (128 mL, 128 mmol) was added to 5-Methoxymethyl-isophthalic acid dimethyl ester (32g, 134.3 mmol) in MeOH (650 mL) and the mixture was stirred at r.t. over night. The mixture was evaporated and the residue was portioned between water and EtOAc. 10% HCl was added to the water phase until pH 1 and this was extracted with EtOAC, the organic phase was dried ($MgSO_4$), filtered and concentrated to give 28g of the title compound as a white solid.
$^1$H NMR: 3.46 (s, 3 H), 3.97 (s, 3 H), 4.58 (s, 2 H), 8.28 (s, 2 H), 8.70 (s, 1 H)
$^{13}$C NMR: 52.4, 58.5, 73.5, 129.9, 130.6, 131.0, 133.2, 133.6, 139.6, 166.0, 170.7

**5-Methoxymethyl-isophthalic acid dimethyl ester**

5-Bromomethyl-isophthalic acid dimethyl ester (44g, 140 mmol), $K_2CO_3$ (43g, 308 mmol), MeOH (350 mL) and THF (350 mL) was heated 60 °C for 2h. The mixture was portioned between $H_2O$ and EtOAC and the organic phase was washed with brine, dried ($MgSO_4$) filtered and concentrated to give 32g of the title compound as a white solid.
$^1$H NNMR: 3.43 (s, 3 H), 3.96 (s, 6 H), 4.55 (s, 2 H), 8.21 (s, 2 H), 8.61 (s, 1 H)
$^{13}$C NMR: 52.8, 58.9, 74.0, 130.4, 131.2, 133.2, 140.0, 166.6

**5-Bromomethyl-isophthalic acid dimethyl ester**

N Bromosuccinimide (45g, 238 mmol) and triphenylphosphine (65g, 238 mmol) was added to 5-Hydroxymethyl-isophthalic acid dimethyl ester (40g, 159 mmol) in $CH_2Cl_2$ (700 mL) at 0 °C. The mixture was stirred at 0°C for 1 h and then diluted with $CH_2Cl_2$ (700 mL). The mixture was washed with saturated $NaHCO_3$ followed by brine and the organic phase was dried ($MgSO_4$) filtered and concentrated. The crude was purified by flash column chromatography over silica gel, eluting with heptane to heptanelEtOAc (4:1) to give 44.5 g of the title compound as a white solid.
$^1$H NMR 1.43 (t, 6 H), 4.43 (q, 4 H), 4.56 (s, 2 H), 8.25 (d, 2 H), 8.61 (t, 1H)
$^{13}$C NMR: 14.3, 31.6, 61.6, 130.5, 131.6, 134.0, 138.7, 165.3

**Abbreviations**

| | |
|---|---|
| DIBAL | diisobutylaluminium hydride |
| THF | tetrahydrofurane |
| DMF | N,N-dimethylformamide |
| DMA | N,N-dimethylacetamide |
| DMSO | dimethylsulphoxide |
| HOAc | acetic acid |
| *n*BuLi | n-butyllithium |
| MeOH | methanol |
| Ph | phenyl |
| *t*BuOH | t-butanol |

| | |
|---|---|
| BOC | tert-butoxycarbonyl |
| EtOAc | ethyl acetate |
| AcOOH | peracetic acid |
| mCPBA | 3-chloroperoxybenzoic acid |
| $Ac_2O$ | acetic anhydride |
| Ac | acetate |
| dba | dibenzylideneacetone |
| BINAP | 2,2'-bis(diphenylphosphino)-1,1'-binaphtyl |
| DPPF | 1, 1' -bis(diphenylphosphino)ferrocene |
| TfOH | trifluoromethanesulphonic acid |
| ppm | parts per million |

**Pharmacology**

The pharmacological properties of the compounds of the invention can be analyzed using standard assays for functional activity. Examples of glutamate receptor assays are well known in the art as described in for example Aramori et al., Neuron 8:757 (1992), Tanabe et al., Neuron 8:169 (1992), Miller et al., J. Neuroscience 15: 6103 (1995), Balazs, et al., J. Neurochemistry 69:151 (1997). The methodology described in these publications is incorporated herein by reference. Conveniently, the compounds of the invention can be studied by means of an assay that measures the mobilization of intracellular calcium, $[Ca^{2+}]_i$ in cells expressing mGluR5.
For FLIPR analysis, cells expressing human mGluR5d or recombinant mGluR1 as described in WO 97/05252 were seeded on collagen coated clear bottom 96-well plates with black sides and analysis of $[Ca^{2+}]_i$ mobilization was done 24 h after seeding.

FLIPR experiments were done using a laser setting of 0.800 W and a 0.4 second CCD camera shutter speed. Each FLIPR experiment was initiated with 160 μl of buffer present in each well of the cell plate. After each addition of the compound, the fluorescence signal was sampled 50 times at 1 second intervals followed by 3 samples at 5 second intervals. Responses were measured as the peak height of the response within the sample period. $EC_{50}$ and $IC_{50}$ determinations were made from data obtained from 8-point concentration response curves (CRC) performed in duplicate. Agonist CRC were generated by scaling all responses to the maximal response observed for the plate. Antagonist block of the agonist challenge was normalized to the average response of the agonist challenge in 14 control wells on the same plate.

We have validated a secondary functional assay for mGluR5d or recombinant mGluR1 as described in WO 97/05252 based on Inositol Phosphate ($IP_3$) turnover. $IP_3$ accumulation is measured as an index of receptor mediated phospholipase C turnover. GHEK cells stably expressing the human mGluR5d or recombinant mGluR1 receptors were incubated with [3H] myo-inositol overnight, washed three times in HEPES buffered saline and pre-incubated for 10 min with 10 mM LiCl. Compounds (agonists) were added and incubated for 30 min at 37°C. Antagonist activity was determined by pre-incubating test compounds for 15 min, then incubating in the presence of glutamate (80μM) or DHPG (30 μM) for 30 min. Reactions were terminated by the addition of perchloric acid (5%). Samples were collected and neutralized, and inositol phosphates were separated using Gravity-Fed Ion-Exchange Columns.

A detailed protocol for testing the compounds of the invention is provided in the assay below.

**Assay of Group I receptor antagonist activity**

For FLIPR analysis, cells expressing human mGluR5d or recombinant mGluRl as described in WO 97/05252 were seeded on collagen coated clear bottom 96-well plates with black sides and analysis of $[Ca^{2+}]_i$ mobilization was performed 24 h following seeding.
Cell cultures in the 96-well plates were loaded with a 4 μM solution of acetoxymethyl ester form of the fluorescent calcium indicator fluo-3 (Molecular Probes, Eugene, Oregon) in 0.01% pluronic. All assays were performed in a buffer containing 127 mM NaCl, 5 mM KCl, 2 mM $MgCl_2$, 0.7 mM $NaH_2PO_4$, 2 mM $CaCl_2$, 0.422 mg/ml $NaHCO_3$, 2.4 mg/ml HEPES, 1.8 mg/ml glucose and 1 mg/ml BSA Fraction IV (pH 7.4).
FLIPR experiments were done using a laser setting of 0.800 W and a 0.4 second CCD camera shutter speed with excitation and emission wavelengths of 488 nm and 562 nm, respectively. Each FLIPR experiment was initiated with 160 μl of buffer present in each well of the cell plate. A 40 μl addition from the antagonist plate was followed by a 50 μL addition from the agonist plate. After each addition the fluorescence signal was sampled 50 times at 1 second intervals followed by 3 samples at 5 second intervals. Responses were measured as the peak height of the response within the sample period.
$EC_{50}/IC_{50}$ determinations were made from data obtained from 8 points concentration response curves (CRC) performed in duplicate. Agonist CRC were generated by scaling all responses to the maximal response observed for the plate.

Antagonist block of the agonist challenge was normalized to the average response of the agonist challenge in 14 control wells on the same plate.

*Measurement of Inositol Phosphate Turnover in Intact Whole Cells*

GHEK stably expressing the human mGluR5d or recombinant mGluR1 receptor were seeded onto 24 well poly-L-lysine coated plates at 40 x $10^4$ cells /well in media containing 1 μCi/well [3H] myo-inositol. Cells were incubated overnight (16 h), then washed three times and incubated for 1 h at 37°C in HEPES buffered saline (146 mM NaCl, 4.2 mM KC1,0.5 mM $MgCl_2$, 0.1% glucose, 20 mM HEPES, pH 7.4) supplemented with 1 unit/ml glutamate pyruvate transaminase and 2 mM pyruvate. Cells were washed once in HEPES buffered saline and pre-incubated for 10 min in HEPES buffered saline containing 10 mM LiCl. Compounds (agonists) were added and incubated at 37°C for 30 min. Antagonist activity was determined by pre-incubating test compounds for 15 min, then incubating in the presence of glutamate (80 μM) or DHPG (30 μM) for 30 min. The reaction was terminated by the addition of 0.5 ml perchloric acid (5%) on ice, with incubation at 4°C for at least 30 min. Samples were collected in 15 ml Falcon tubes and inositol phosphates were separated using Dowex columns, as described below.

*Assay For Inositol Phosphates Using Gravity-Fed Ion-Exchange Columns*

Preparation of Ion- Exchange Columns

Ion-exchange resin (Dowex AG1-X8 formate form, 200-400 mesh, BIORAD) was washed three times with distilled water and stored at 4°C. 1.6 ml resin was added to each column, and washed with 3 ml 2.5 mM HEPES, 0.5 mM EDTA, pH 7.4.

a) Sample Treatment

Samples were collected in 15 ml Falcon tubes and neutralized with 0.375 M HEPES, 0.75 M KOH. 4 ml of HEPES / EDTA (2.5 / 0.5 mM, pH 7.4) were added to precipitate the potassium perchlorate. Supernatant was added to the prepared Dowex columns.

b) Inositol Phosphate Separation

Elute glycero phosphatidyl inositols with 8 ml 30 mM ammonium formate.
Elute total inositol phosphates with 8 ml 700 mM ammonium formate / 100 mM formic acid and collect eluate in scintillation vials. Count eluate mixed with 8 ml scintillant.
One aspect of the invention relates to a method for inhibiting activation of mGluR5 receptors, comprising treating a cell containing said receptor with an effective amount of the compound of formula I.

**Screening for compounds active against TLESR**

Adult Labrador retrievers of both genders, trained to stand in a Pavlov sling, are used. Mucosa-to-skin esophagostomies are formed and the dogs are allowed to recover completely before any experiments are done.

*Motility measurement*

In brief, after fasting for approximately 17 h with free supply of water, a multilumen sleeve/sidehole assembly (Dentsleeve, Adelaide, South Australia) is introduced through the esophagostomy to measure gastric, lower esophageal sphincter (LES) and esophageal pressures. The assembly is perfused with water using a low-compliance manometric perfusion pump (Dentsleeve, Adelaide, South Australia). An air-perfused tube is passed in the oral direction to measure swallows, and an antimony electrode monitored pH, 3 cm above the LES. All signals are amplified and acquired on a personal computer at 10 Hz.
When a baseline measurement free from fasting gastric/LES phase III motor activity has been obtained, placebo (0.9% NaCl) or test compound is administered intravenously (i.v., 0.5 ml/kg) in a foreleg vein. Ten min after i.v. administration, a nutrient meal (10% peptone, 5% D-glucose, 5% Intralipid, pH 3.0) is infused into the stomach through the central lumen of the assembly at 100 ml/min to a final volume of 30 ml/kg. The infusion of the nutrient meal is followed by air infusion at a rate of 500 ml/min until an intragastric pressure of 10±1 mmHg is obtained. The pressure is then maintained at this level throughout the experiment using the infusion pump for further air infusion or for venting air from the stomach. The experimental time from start of nutrient infusion to end of air insufflation is 45 min. The procedure has

been validated as a reliable means of triggering TLESRs.

TLESRs is defined as a decrease in lower esophageal sphincter pressure (with reference to intragastric pressure) at a rate of >1 mmHg/s. The relaxation should not be preceded by a pharyngeal signal ≤2s before its onset in which case the relaxation is classified as swallow-induced. The pressure difference between the LES and the stomach should be less than 2 mmHg, and the duration of the complete relaxation longer than 1 s.

**Abbreviations**

| | |
|---|---|
| BSA | Bovine Serum Albumin |
| CCD | Charge Coupled Device |
| CRC | Concentration Response Curve |
| DHPG | 3,5-dihydroxyphenylglycine; |
| EDTA | Ethylene Diamine Tetraacetic Acid |
| FLIPR | Fluorometric Imaging Plate reader |
| GHEK | GLAST-containing Human Embrionic Kidney |
| GLAST | glutamate/aspartate transporter |
| HEPES | 4-(2-hydroxyethyl)-1-piperazineethanesulfonic acid (buffer) |
| $IP_3$ | inositol triphosphate |

**Metabolic stability**

The compounds were incubated (at 1 μM) with characterized, pooled human liver microsomes (0.5 mg microsomal protein/mL incubation) at 37°C in phosphate buffer (50 mM potassium phosphate, pH 7.4) The incubations were started by the addition of a cofactor (1 mM NADPH (β-Nicotinamide adenine dinucleotide phosphate in reduced form) and terminated by the addition of acetonitrile (1:1).

The incubations were analyzed by LC/MS and a time curve for the disappearance of the parent compound was constructed. Control incubations without microsomes, or with acetonitrile addition before the addition of the cofactor, were used to check for non-enzymatic degradation/disappearance.

The intrinsic clearance of the liver was calculated from the halflife (in minutes) in the incubations according to the following equation:

$$CL_{\text{int}} = \frac{0.693}{T_{1/2}} \bullet \frac{\text{mL incubation}}{\text{mg microsomes}} \bullet \frac{\text{x mg microsomes}}{\text{g liver}} \bullet \frac{\text{y g liver}}{\text{human(Bw)}}$$

where x was set to be 50 (mg microsomal protein per g liver) and y was set to be 1500 (g liver) and the body weight, Bw, was set to be 70 kg. The hepatic clearance due to metabolism, $CL_H$, was calculated from the intrinsic clearance, $CL_{int}$, according to the following equation:

$$CL_H = \frac{Q_H \bullet f_u \bullet CL_{\text{int}}}{Q_H + f_u \bullet CL_{\text{int}}}$$

using 1450 mL/min as the liver blood flow, $Q_H$, and using $f_u$=1 thus disregarding protein binding. The fraction escaping hepatic clearance, $F_H$, given in %, was calculated from the hepatic clearance according to the following equation:

$$F_H = (1 - \frac{CL_H}{Q_H}) \bullet 100 = (1 - \frac{f_u \bullet CL_{\text{int}}}{Q_H + f_u \bullet CL_{\text{int}}}) \bullet 100$$

Thus, the higher the $F_H$, the more metabolically stable the compound.

**Solubility**

A detailed protocol for determining the solubility of the compounds of the invention, and other related compounds herein, is provided below.

Duplicate of 1-2 mg of the test compound are incubated in 0.1 M phosphate buffer, pH 7.4, in 2 mL glass-vials on a plate bed shaker (IKA®-Schüttler MTS-4, IKA Labortechnik) at 300 rpm at a temperature of 20 to 22 °C for 24 hours. Non-dissolved material is centrifugated down from the saturated solution at 3000 rpm for 2 x 15 minutes at 22 °C (Multifuge® 3 S-R, Heraeus). The aqueous supernatant is transferred to glass-vials and aliquotes used for quantitative analysis using reversed HPLC with MS confirmation of compound identity. Quantification is performed by the UV-trace of the chromatogram against a calibration curve, 0-200 μM, of the test compound dissolved in DMSO at the wavelength of a positive inflection point of the UV spectrum. The reported values (aq. solubility) are the mean values of duplicate HPLC-analysis of two independent incubations.

**Results**

Typical $IC_{50}$ values as measured in the FLIPR assays described above are 10 μM or less. In one aspect of the invention the $IC_{50}$ is below 2 μM. In another aspect of the invention the $IC_{50}$ is below 0.2 μM. In a further aspect of the invention the $IC_{50}$ is below 0.05 μM.

A comparative study on the variation of the Z-substituent in the structure of formula XII revealed an unexpected importance of the nature of this substituent considering simultaneously the potency at the mGluR5 receptor (FLIPR analysis), the human microsomal metabolic stability (h. mic. $F_H$), and the aqueous solubility (aq. solubility) of the corresponding analogs. For example, a 21-fold increase in potency, and a 22-fold increase in solubility, was observed for 3-fluoro-5-[5-(5-fluoropyridin-2-yl)-2*H*-tetrazol-2-yl]benzonitrile (Z=F) when compared to the corresponding bromo derivative 3-[5-(5-bromopyridin-2-yl)-2*H* tetrazol-2-yl]-5-fluorobenzonitrile (Z=Br). Simultaneously, the former is significantly more stable when incubated with human microsomes (h.mic. $F_H$) as compared to the unsubstituted analog 3-fluoro-5-(5-pyridin-2-yl-2*H*-tetrazol-2-yl)benzonitrile (Z=H). The significantly higher human metabolic stability (h.mic. $F_H$) of 3-fluoro-5-[5-(5-fluoropyridin-2-yl)-2*H*-tetrazol-2-yl]benzonitrile (Z=F) and 3-[5-(5-bromopyridin-2-yl)-2*H*-tetrazol-2-yl]-5-fluorobenzonitrile (Z=Br) as compared to 3-fluoro-5-(5-pyridin-2-yl-2*H*-tetrazol-2-yl)benzonitrile (Z=H) indicates a necessity for compounds of formula I to carry a substituent Z other than hydrogen to withhold acceptable human metabolic stability. Fluoro, chloro and cyano as Z substituent in compounds of formula I constitutes an improvement, when considering multiple parameters of importance for the suitability as a drug product targeting the mGluR5 receptor, as compared to known compounds of formula I in which Z is else but fluoro, chloro or cyano.

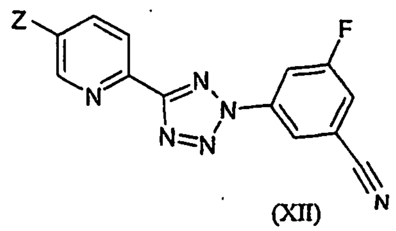

(XII)

| Compound | Z | IC50 mGluR5 (nM) | aq. Solubility (uM) | h.mic. $F_H$ (%) |
|---|---|---|---|---|
| 3-Fluoro-5-[5-(5-fluoropyridin-2-yl)-2*H*-tetrazol-2-yl] benzonitrile | F | 3 | 4.3 | 71 |
| 6-[2-(3-Cyano-5-fluorophenyl)-2*H*-tetrazol-5-yl] mcotmonitrile[1)] | CN | 9 | - | - |
| 3-Fluoro-5-(5-pyridin-2-yl-2*H* tetrazol-2-yl)benzonitrile | H | 12 | 43 | 10 |
| 3-[5-(5-Chloropyridin-2-yl)-2*H*-tetrazol-2-yl]-5-fluorobenzonitrile | Cl | 18 | - | - |

(continued)

| Compound | Z | IC50 mGluR5 (nM) | aq. Solubility (uM) | h.mic. $F_H$ (%) |
|---|---|---|---|---|
| 3-[5-(5-Bromopyridin-2-yl)-2*H*-tetrazol-2-yl]-5-fluorobenzonitrile[2] | Br | 62 | 0.2 | 92 |

[1] Example 116 of WO 03/029210
[2] Example 62 of WO 03/029210

## Claims

1. Compounds of formula I

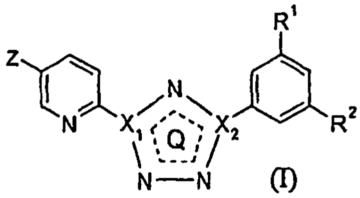

(I)

wherein:

$X_1$ is N and $X_2$ is C or $X_1$ is C and $X_2$ is N;
Z is fluoro or chloro;
$R^1$ and $R^2$ are independently selected from the group consisting of hydrogen, hydroxy, halo, $C_{1-6}$alkylhalo, $OC_{1-6}$alkylhalo, $C_{1-6}$alkyl, $OC_{0-6}$alkyl, $C_{1-6}$alkyl$OR^4$, $OC_{2-6}$alkyl$OR^4$, $C_{0-6}$alkylcyano, $C_{0-6}$alkyl$NR^4R^5$ and $OC_{2-6}$alkyl$NR^4R^5$;
$R^4$ and $R^5$ are independently selected from the group consisting of hydrogen, hydroxy and $C_{1-3}$alkyl;

or salts, solvates or solvated salts thereof.

2. The compounds of claim 1 wherein $X_1$ is C and $X_2$ is N.

3. The compounds according to any one of the preceeding claims wherein Z is fluoro.

4. The compounds according to any one of the preceeding claims wherein $R^1$ and $R^2$ are selected from the group consisting of hydrogen, hydroxy, halo, $-C_{1-3}$alkylhalo, $-OC_{1-3}$alkylhalo, $-C_{1-3}$alkyl, $-OC_{0-3}$alkyl, $-C_{1-3}$alkyl$OR^4$, $-OC_{2-4}$alkyl$OR^4$ $-C_{0-3}$alkylcyano and $C_{0-3}$alkyl$NR^4R^5$; and $R^4$ and $R^5$ are independently selected from hydrogen, methyl and ethyl.

5. The compounds according to any one of the preceeding claims wherein $R^1$ is fluoro, chloro, bromo, iodo, methoxymethyl, methoxy, difluoromethoxy, trifluoromethoxy, 2-methoxy-ethoxy, ethylamino or amine.

6. The compounds according to any one of the preceeding claims wherein $R^2$ is fluoro or cyano.

7. A Compound according to claim 1, said compound being selected from the group consisting of

3-fluoro-5-[5-(5-fluoropyridin-2-yl)-2H-tetrazol-2-yl]benzonitrile,
3-[5-(5-chloropyridin-2-yl)-2H-tetrazol-2-yl]-5-fluorobenzonitrile,
3-[5-(5-fluoro-pyridin-2-yl)-tetrazol-2-yl]-5-methoxymethyl-benzonitrile,
3-fluoro-5-[2-(5-fluoropyridin-2-yl)-2H-tetrazol-5-yl]benzonitrile,
3-[2-(5-chloropyridin-2-yl)-2H-tetrazol-5-yl]-5-fluorobenzonitrile,
3-[5-(5-fluoropyridin-2-yl)-2H-tetrazol-2-yl]-5-(methoxymethyl)benzonitrile,
5-fluoro-2-[2-(3-fluoro-5.methoxyphenyl)-2H-tetrazol-5-yl]pyridine,
3-[5-(5-fluoro-pyridin-2-yl)-2H-tetrazol-2-yl]-5-methoxybenzonitrile,

3-[5-(5-fluoropyridin-2-yl)-2H-tetrazol-2-yl]-5.(trifluoromethoxy)benzonitrile,
3-(difluoromethoxy)-5-[5-(5-fluoropyridin-2-yl)-2H-tetrazol-2-yl]benzonitrile,
3-[5-(5-fluoropyridin-2-yl)-2H-tetrazol-2-yl]-5-(2-methoxyethoxy)benzonitrile,
3-(ethylamino)-5-[5-(5-fluoropyridin-2-yl)-2H-tetrazol-2-yl]benzonitrile,
3-amino-5-[5-(5-fluoropyridin-2-yl)-2H-tetrazol-2-yl]benzonitrile,
3-[5-(5-fluoropyridiii-2-yl)-2H-tetrazol-2-yl]-5-iodobenzonitrile, and

salts, solvates or solvated salts thereof.

**8.** A pharmaceutical composition comprising as active ingredient a therapeutically effective amount of the compound according to any one of claims 1 to 7, in association with one or more pharmaceutically acceptable diluent, excipients and/or inert carrier.

**9.** The pharmaceutical composition according to claim 8, for use in the treatment of mGluR5 receptor mediated disorders.

**10.** The compound according to any one of claims 1 to 7, for use in therapy.

**11.** The compound according to any of claims 1 to 7, for use in the treatment of neurological disorders, psychiatric disorders, pain disorders or gastrointestinal disorders.

**12.** Use of a compound according to any of claims 1 to 7, in the manufacture of a medicament for the treatment of neurological disorders, psychiatric disorders, pain disorders or gastrointestinal disorders.

**13.** The use according to claim 12, where the medicament manufactured is for the treatment of neurological disorders.

**14.** The use according to claim 12, where the medicament manufactured is for the treatment of psychiatric disorders.

**15.** The use according to claim 12, where the medicament manufactured is for the treatment of chronic and acute pain disorders.

**16.** The use according to claim 12, where the medicament manufactured is for the treatment of gastrointestinal disorders.

**17.** The use according to claim 13 or 14, where the medicament manufactured is for the treatment of Alzheimer's disease senile dementia, AIDS-induced dementia, Parkinson's disease, amylotropic lateral sclerosis, Huntington's Chorea, migraine, epilepsy, schizophrenia, depression, anxiety, acute anxiety, ophthalmological disorders such as retinopathies, diabetic retinopathies, glaucoma, auditory neuropathic disorders such as tinnitus, chemotherapy induced neuropathies, post-herpetic neuralgia and trigeminal neuralgia, tolerance, dependency, Fragile X, autism, mental retardation, Down's Syndrome, stroke, head trauma, anoxic and ischemic injuries, hypoglycemia and cardiovascular diseases.

**18.** The use according to claim 15, where the medicament manufactured is for the treatment of pain related to migraine, inflammatory pain, neuropathic pain disorders such as diabetic neuropathies, arthritis and rheumatitiod diseases, low back pain, post-operative pain and pain associated with various conditions including angina, renal or billiary colic, menstruation, migraine and gout.

**19.** Use of a compound according to any of claims 1 to 7, in the manufacture of a medicament for the inhibition of transient lower esophageal sphincter relaxations, for the treatment of GERD, for the prevention of reflux, for the treatment regurgitation, treatment of asthma, treatment of laryngitis, treatment of lung disease and for the management of failure to thrive.

**20.** An *in vitro* method for inhibiting activation of mGluR5 receptors, comprising treating a cell containing said receptor with an effective amount of the compound according to claim 1.

**Patentansprüche**

**1.** Verbindungen der Formel I

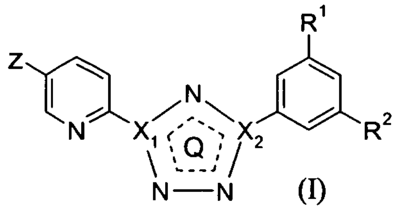

(I)

worin:

$X_1$ N ist und $X_2$ C ist oder $X_1$ C ist und $X_2$ N ist;
Z Fluor oder Chlor ist;
$R^1$ und $R^2$ unabhängig voneinander ausgewählt sind aus der Gruppe, bestehend aus Wasserstoff, Hydroxy, Halogen, $C_{1-6}$-Alkylhalogen, $OC_{1-6}$-Alkylhalogen, $C_{1-6}$-Alkyl, $OC_{0-6}$-Alkyl, $C_{1-6}$-Alkyl-$OR^4$, $OC_{2-6}$-Alkyl-$OR^4$, $C_{0-6}$-Alkylcyano, $C_{0-6}$-Alkyl-$NR^4R^5$ und $OC_{2-6}$-Alkyl-$NR^4R^5$;
$R^4$ und $R^5$ unabhängig voneinander ausgewählt sind aus der Gruppe, bestehend aus Wasserstoff, Hydroxy und $C_{1-3}$-Alkyl;

Salze, Solvate oder solvatisierte Salze davon.

**2.** Verbindungen nach Anspruch 1, worin $X_1$ C ist und $X_2$ N ist.

**3.** Verbindungen nach einem der vorhergehenden Ansprüche, worin Z Fluor ist.

**4.** Verbindungen nach einem der vorhergehenden Ansprüche, worin $R^1$ und $R^2$ ausgewählt sind aus der Gruppe, bestehend aus Wasserstoff, Hydroxy, Halogen, -$C_{1-3}$-Alkylhalogen, -$OC_{1-3}$-Alkylhalogen, -$C_{1-3}$-Alkyl, -$OC_{0-3}$-Alkyl, -$C_{1-3}$Alkyl-$OR^4$, -$OC_{2-4}$-Alkyl-$OR^4$, $C_{0-3}$-Alkylcyano und -$C_{0-3}$-Alkyl-$NR^4R^5$; und $R^4$ und $R^5$ unabhängig voneinander ausgewählt sind aus Wasserstoff, Methyl und Ethyl.

**5.** Verbindungen nach einem der vorhergehenden Ansprüche, worin $R^1$ Fluor, Chlor, Brom, Iod, Methoxymethyl, Methoxy, Difluormethoxy, Trifluormethoxy, 2-Methoxy-ethoxy, Ethylamino oder Amin ist.

**6.** Verbindungen nach einem der vorhergehenden Ansprüche, worin $R^2$ Fluor oder Cyano ist.

**7.** Verbindung nach Anspruch 1, worin die Verbindung ausgewählt ist aus der Gruppe, bestehend aus:

3-Fluor-5-[5-(5-fluorpyridin-2-yl)-2H-tetrazol-2-yl]benzonitril,
3-[5-(5-Chlorpyridin-2-yl)-2H-tetrazol-2-yl]-5-fluorbenzonitril,
3-[5-(5-Fluorpyridin-2-yl)-tetrazol-2-yl]-5-methoxymethylbenzonitril,
3-Fluor-5-[2-(5-fluorpyridin-2-yl)-2H-tetrazol-5-yl]benzonitril,
3-[2-(5-Chlorpyridin-2-yl)-2H-tetrazol-5-yl]-5-fluorbenzonitril,
3-[5-(5-Fluorpyridin-2-yl)-2H-tetrazol-2-yl]-5-(methoxymethyl)benzonitril,
5-Fluor-2-[2-(3-fluor-5-methoxyphenyl)-2H-tetrazol-5-yl]pyridin,
3-[5-(5-Fluorpyridin-2-yl)-2H-tetrazol-2-yl]-5-methoxybenzonitril,
3-[5-(5-Fluorpyridin-2-yl)-2H-tetrazol-Z-yl]-5-(trifluormethoxy)benzonitril,
3-(Difluormethoxy)-5-[5-(5-fluorpyridin-2-yl)-2H-tetrazol-2-yl]benzonitril,
3-[5-(5-Fluorpyridin-2-yl)-2H-tetrazol-2-yl]-5-(2-methoxyethoxy)benzonitril,
3-(Ethylamino)-5-[5-(5-fluorpyridin-2-yl)-2H-tetrazol-2-yl]benzonitril,
3-Amino-5-[5-(5-fluorpyridin-2-yl)-2H-tetrazol-2-yl]benzonitril,
3-[5-(5-Fluorpyridin-2-yl)-2H-tetrazol-2-yl]-5-iodbenzonitril und

Salze, Solvate oder solvatisierte Salze davon.

**8.** Pharmazeutische Zusammensetzung, die als Wirkstoff eine therapeutisch wirksame Menge der Verbindung nach einem der Ansprüche 1 bis 7 zusammen mit einem oder mehreren pharmazeutisch unbedenklichen Verdünnungsmitteln, Hilfsmitteln und/oder inerten Trägern umfasst.

9. Pharmazeutische Zusammensetzungen nach Anspruch 8 zur Verwendung bei der Behandlung von Störungen, die über den mGluR5-Rezeptor vermittelt werden.

10. Verbindung nach einem der Ansprüche 1 bis 7 zur Verwendung in der Therapie.

11. Verbindung nach einem der Ansprüche 1 bis 7 zur Verwendung bei der Behandlung von neurologischen Störungen, psychiatrischen Störungen, Schmerzstörungen oder gastrointestinalen Störungen.

12. Verwendung einer Verbindung nach einem der Ansprüche 1 bis 7 bei der Herstellung eines Arzneimittels zur Behandlung von neurologischen Störungen, psychiatrischen Störungen, Schmerzstörungen oder gastrointestinalen Störungen.

13. Verwendung nach Anspruch 12, wobei das hergestellte Arzneimittel zur Behandlung von neurologischen Störungen dient.

14. Verwendung nach Anspruch 12, wobei das hergestellte Arzneimittel zur Behandlung von psychiatrischen Störungen dient.

15. Verwendung nach Anspruch 12, wobei das hergestellte Arzneimittel zur Behandlung von chronischen und akuten Schmerzstörungen dient.

16. Verwendung nach Anspruch 12, wobei das hergestellte Arzneimittel zur Behandlung von gastrointestinalen Störungen dient.

17. Verwendung nach Anspruch 13 oder 14, wobei das hergestellte Arzneimittel zur Behandlung von durch die Alzheimer-Krankheit verursachter seniler Demenz, durch AIDS induzierter Demenz, Morbus Parkinson, amyotrophischer Lateralsklerose, Huntington-Chorea, Migräne, Epilepsie, Schizophrenie, Depression, Angst, akuten Angstzuständen, ophthalmologischen Störungen, wie Retinopathien, diabetischen Retinopathien, Glaukom, auditorischen neuropathischen Störungen, wie Tinnitus, durch Chemotherapie induzierten Neuropathien, nach Herpes auftretender Neuralgie und Trigeminusneuralgie, Toleranz, Abhängigkeit, Syndrom des fragilen X-Chromosoms, Autismus, Intelligenzminderung, Down-Syndrom, Schlaganfall, Schädeltrauma, anoxischen und ischämischen Läsionen, Hypoglykämie und Herz-Kreislaufkrankheiten dient.

18. Verwendung nach Anspruch 15, wobei das hergestellte Arzneimittel zur Behandlung von Schmerzen in Verbindung mit Migräne, Entzündungsschmerzen, neuropathischen Schmerzstörungen, wie diabetischen Neuropathien, Arthritis und rheumatoiden Erkrankungen, Lendenschmerzen, postoperativen Schmerzen und Schmerzen, die mit verschiedenen Zuständen verbunden sind, einschließlich Angina, Nieren- oder Gallenkolik, Menstruation, Migräne und Gicht, dient.

19. Verwendung einer Verbindung nach einem der Ansprüche 1 bis 7 zur Herstellung eines Arzneimittels zur Hemmung der vorübergehenden Entspannung des unteren Speiseröhren-Schließmuskels, zur Behandlung von GERD, zur Prävention von Reflux, zur Behandlung von Regurgitation, zur Behandlung von Asthma, zur Behandlung von Laryngitis, zur Behandlung von Lungenkrankheiten und zur Behandlung von Gedeihstörungen.

20. In-vitro-Verfahren zur Hemmung der Aktivierung von mGluR5-Rezeptoren, die umfassend die Behandlung einer Zelle, die diesen Rezeptor enthält, mit einer wirksamen Menge einer Verbindung nach Anspruch 1.

**Revendications**

1. Composés de formule I

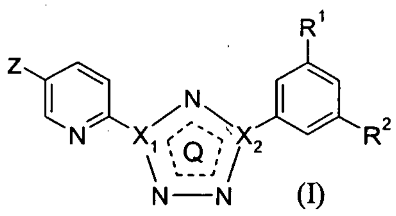

(I)

dans laquelle :

$X_1$ est N et $X_2$ est C ou $X_1$ est C et $X_2$ est N ;
Z est fluoro ou chloro ;
$R^1$ et $R^2$ sont choisis indépendamment dans le groupe consistant en hydrogène, hydroxyle, halo, $C_{1-6}$alkylhalo, $OC_{1-6}$alkylhalo, $C_{1-6}$alkyle, $OC_{0-6}$alkyle, $C_{1-6}$alkylOR$^4$ $OC_{2-6}$alkylOR$^4$, $C_{0-6}$alkylcyano, $C_{0-6}$alkylNR$^4$R$^5$ et $OC_{2-6}$alkylNR$^4$R$^5$;
$R^4$ et $R^5$ sont choisis indépendamment dans le groupe consistant en hydrogène, hydroxyle et $C_{1-3}$alkyle ;

ou des sels, des solvates ou des sels solvatés de ceux-ci.

**2.** Composés de la revendication 1, dans lesquels $X_1$ est C et $X_2$ est N.

**3.** Composés selon l'une quelconque des revendications précédentes, dans lesquels Z est fluoro.

**4.** Composés selon l'une quelconque des revendications précédentes, dans lesquels $R^1$ et $R^2$ sont choisis dans le groupe consistant en hydrogène, hydroxyle, halo, -$C_{1-3}$alkylhalo, -$OC_{1-3}$alkylhalo, -$C_{1-3}$alkyle, -$OC_{0-3}$alkyle, -$C_{1-3}$alkylOR$^4$, -$OC_{2-4}$alkylOR$^4$,
-$C_{0-3}$alkylcyano et -$C_{0-3}$alkylNR$^4$R-$^5$ ; et $R^4$ et $R^5$ sont choisis indépendamment parmi hydrogène, méthyle et éthyle.

**5.** Composés selon l'une quelconque des revendications précédentes, dans lesquels $R^1$ est fluoro, chloro, bromo, iodo, méthoxyméthyle, méthoxy, difluorométhoxy, trifluorométhoxy, 2-méthoxy-éthoxy, éthylamino ou amine.

**6.** Composés selon l'une quelconque des revendications précédentes, dans lesquels $R^2$ est fluoro ou cyano.

**7.** Composé selon la revendication 1, ledit composé étant choisi dans le groupe consistant en :

3-fluoro-5-[5-(5-fluoropyridin-2-yl)-2H-tétrazol-2-yl]benzonitrile,
3-[5-(5-chloropyridin-2-yl)-2H-tétrazol-2-yl]-5-fluorobenzonitrile,
3-[5-(5-fluoro-pyridin-2-yl)-tétrazol-2-yl]-5-méthoxyméthyl-benzonitrile,
3-fluoro-5-[2-(5-fluoropyridin-2-yl)-2H-tétrazol-5-yl]benzonitrile,
3-[2-(5-chloropyridin-2-yl)-2H-tétrazol-5-yl]-5-fluorobenzonitrite,
3-[5-(5-fluoropyridin-2-yl)-ZH-tétrazol-2-yl]-5-(méthoxyméthyl)benzonitrile,
5-fluoro-2-[2-(3-fluoro-5-méthoxyphényl)-2H-tétrazol-5-yl]pyridine,
3-[5-(5-fluoro-pyridin-2-yl)-2H-tétrazol-2-yl]-5-méthoxybenzonitrile,
3-[5-(5-fluoropyridin-2-yl)-2H-tétrazol-2-yl]-5-(trifluorométhoxy)benzonitrile,
3-(difluorométhoxy)-5-[5-(5-fluoropyridin-2-yl)-2H-tétrazol-2-yl]benzonitrile,
3-[5-(5-fluoropyridin-2-yl)-2H-tétrazol-2-yl]-5-(2-méthoxyéthoxy)benzonitrile,
3-(éthylamino)-5-[5-(5-fluoropyridin-2-yl)-2H-tétrazol-2-yl]benzonitrile,
3-amino-5-[5-(5-fluoropyridin-2-yl)-2H-tétrazol-2-yl]benzonitrile, et
3-[5-(5-fluoropyridin-2-yl)-2H-tétrazol-2-yl]-5-iodobenzonitrile,

et des sels, des solvates ou des sels solvatés de celui-ci.

**8.** Composition pharmaceutique comprenant comme principe actif une quantité thérapeutiquement efficace d'un composé selon l'une quelconque des revendications 1 à 7, en association avec un ou plusieurs solvants, excipients et/ou supports inertes de qualité pharmaceutique.

**9.** Composition pharmaceutique selon la revendication 8, destinée au traitement des troubles induits par le récepteur

mGluR5.

**10.** Composé selon l'une quelconque des revendications 1 à 7, destiné à être utilisé à des fins thérapeutiques.

**11.** Composé selon l'une quelconque des revendications 1 à 7, destiné au traitement de troubles neurologiques, de troubles psychiatriques, de troubles douloureux ou de troubles gastro-intestinaux.

**12.** Utilisation d'un composé selon l'une quelconque des revendications 1 à 7 dans la fabrication d'un médicament destiné au traitement de troubles neurologiques, de troubles psychiatriques, de troubles douloureux ou de troubles gastro-intestinaux.

**13.** Utilisation selon la revendication 12, dans laquelle le médicament fabriqué est destiné au traitement de troubles neurologiques.

**14.** Utilisation selon la revendication 12, dans laquelle le médicament fabriqué est destiné au traitement de troubles psychiatriques.

**15.** Utilisation selon la revendication 12, dans laquelle le médicament fabriqué est destiné au traitement de troubles douloureux chroniques ou aigus.

**16.** Utilisation selon la revendication 12, dans laquelle le médicament fabriqué est destiné au traitement de troubles gastro-intestinaux.

**17.** Utilisation selon la revendication 13 ou 14, dans laquelle le médicament fabriqué est destiné au traitement de la démence sénile dans la maladie d'Alzheimer, de la démence associée au SIDA, de la maladie de Parkinson, de la sclérose latérale amyotrophique, de la chorée de Huntington, de la migraine, de l'épilepsie, de la schizophrénie, de la dépression, de l'anxiété, de l'anxiété aiguë, des troubles ophtalmologiques tels que les rétinopathies, des rétinopathies diabétiques, des glaucomes, des neuropathies auditives telles que les acouphènes, des neuropathies induites par la chimiothérapie, des névralgies post-herpétiques et des névralgies faciales, de la tolérance, de la dépendance, du syndrome de l'X fragile, de l'autisme, de la déficience mentale, de la trisomie 21, des accidents vasculaires cérébraux, des traumatismes crâniens, des lésions anoxo-ischémiques, de l'hypoglycémie et des maladies cardiovasculaires.

**18.** Utilisation selon la revendication 15, dans laquelle le médicament fabriqué est destiné au traitement de douleurs migraineuses, de douleurs inflammatoires, de troubles douloureux neuropathiques tels que les neuropathies diabétiques, de l'arthrite et de maladies rhumatismales, de lombalgies, de douleurs postopératoires et de douleurs associées à différents états tels que l'angine de poitrine, les coliques néphrétiques ou biliaires, la menstruation, la migraine et la goutte.

**19.** Utilisation d'un composé selon l'une quelconque des revendications 1 à 7 dans la fabrication d'un médicament destiné à l'inhibition des relaxations transitoires du sphincter inférieur de l'oesophage, au traitement des troubles liés au reflux gastro-oesophagien, à la prévention des reflux, au traitement de la régurgitation, au traitement de l'asthme, au traitement des laryngites, au traitement des maladies pulmonaires et au traitement des retards staturo-pondéraux.

**20.** Méthode *in vitro* d'inhibition de l'activation des récepteurs mGluR5, comprenant le traitement d'une cellule contenant ledit récepteur avec une quantité efficace d'un composé selon la revendication 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03077918 A
- WO 03029210 A
- WO 9705252 A


### Non-patent literature cited in the description


- **SCHOEPP et al.** *Trends Phannacol. Sci.,* 1993, vol. 14, 13
- **SCHOEPP.** *Neurochem. Int.,* 1994, vol. 24, 439
- **PIN et al.** *Neuropharmacology,* 1995, vol. 34, 1
- **BORDI ; UGOLINI.** *Prog. Neurobiol.,* 1999, vol. 59, 55
- **NAKANISHI.** *Neuron,* 1994, vol. 13, 1031
- **KNOPFEL et al.** *J. Med. Chem.,* 1995, vol. 38, 1417
- **PIN et al.** *PNAS,* 1992, vol. 89, 10331
- **MINAKAMI et al.** *BBRC,* 1994, vol. 199, 1136
- **JOLY et al.** *J. Neurosci.,* 1995, vol. 15, 3970
- **BASKYS.** *Trends Pharmacol. Sci.,* 1992, vol. 15, 92
- **WATKINS et al.** *Trends Pharmacol. Sci.,* 1994, vol. 15, 33
- **BASHIR et al.** *Nature,* 1993, vol. 363, 347
- **BORTOLOTTO et al.** *Nature,* 1994, vol. 368, 740
- **AIBA et al.** *Cell,* 1994, vol. 79, 365
- **AIBA et al.** *Cell,* 1994, vol. 79, 377
- **MELLER et al.** *Neuroreport,* 1993, vol. 4, 879
- **BORDI ; UGOLINI.** *Brain Res.,* 1999, vol. 871, 223
- **NAKANISHI.** *Neuron,* 1994, vol. 13, 1.031
- **PIN et al.** *Neuropharmacology,* vol. 34, 1
- **SCHOEPP et al.** *Trends Pharmacol. Sci.,* 1993, vol. 14, 13
- **CUNNINGHAM et al.** *Life Sci.,* 1994, vol. 54, 135
- **HOLLMAN et al.** *Ann. Rev. Neurosci.,* 1994, vol. 17, 31
- **SPOOREN et al.** *Trends Pharmacol. Sci.,* 2001, vol. 22, 331
- **GASPARINI et al.** *Curr. Opin. Pharmacol.,* 2002, vol. 2, 43
- **NEUGEBAUER.** *Pain,* 2002, vol. 98
- **HOLLOWAY ; DENT.** *Gastroenterol. Clin. N. Amer.,* 1990, vol. 19, 517-535
- **MITTAL, R.K ; HOLLOWAY, R.H. ; PENAGINI, R. ; BLACKSHAW, L.A. ; DENT, J.** Transient lower esophageal sphincter relaxation. *Gastroenterology,* 1995, vol. 109, 601-610
- **VAN HEERWARDEN, M.A. ; SMOUT A.J.P.M.,.** Diagnosis of reflux disease. *Baillière's Clin. Gastroenterol.,* 2000, vol. 14, 759-774
- **T.W. GREEN ; P.G.M. WUTS.** Protective Groups in Organic Synthesis. Wiley-Interscience, 1999
- Advanced Organic Chemistry. McGraw Hill, 1992
- Organic Synthesis. McGraw Hill, 1994
- *J. Am. Chem. Soc.,* 1983, 7175-7176
- *Tetrahedron Letters,* 2000, 4335-4338
- *J. Org. Chem.,* 2001, 6775-6786
- *Tetrahedron Letters,* 1997, (6367-6370
- *J. Org. Chem.,* 2001, 7729-7737
- *Helv.Chim.Acta,* 1985, 1283-1300
- *J. Med. Chem.,* 1980, 631-634
- *Monatshefte fuer Chemie,* 2001, 403-406
- *Tetrahedron Letters,* 2002, 6221-6223
- *Tetrahedron Letters,* 1998, 2941-2944
- *Tetrahedron Lett,* 1999, 2747-2748
- *J. Org. Chem.,* 2001, 7945-7950
- *J. Org. Chem.,* 2000, 7984-7989
- *J. Org. Chem.,* 1993, 4139-4141
- **ARAMORI et al.** *Neuron,* 1992, vol. 8, 757
- **TANABE et al.** *Neuron,* 1992, vol. 8, 169
- **MILLER et al.** *J. Neuroscience,* 1995, vol. 15, 6103
- **BALAZS et al.** *J. Neurochemistry,* 1997, vol. 69, 151